# EUROPEAN PATENT APPLICATION

(11) **EP 2 409 660 A1**
(43) Date of publication of application: **25.01.2012**
(21) Application number: 11170056.3
(22) Date of filing: 15.06.2011
(51) Int. Cl.: A61B 18/10

(54) **Tissue fusion system and method for performing a self test**

(30) Priority: 23.07.2010 US 842606
(71) Applicant: CONMED CORPORATION, Utica, NY 13502-5994 (US)
(72) Inventor: Stuebe, Brian C., Broomfield, Colorado 80020 (US); Thomson, Richard K., Centennial, Colorado 80111 (US)
(74) Representative: Vuillermoz, Bruno

(57) **Abstract**

An energy source of a thermal tissue operating system performs self-tests using simulation heating elements and jaw heating elements in tissue-grasping jaws of a handpiece. Power is supplied to the simulation heating elements and verified, without necessitating connection of a handpiece. Power is also delivered and verified to each of the two jaw heating elements when the handpiece is connected. Voltage and current are measured by peak hold detectors, and the peak hold detectors are tested to verify correct operation. All tests may be combined in a power on self-test (POST).

## Description

### Cross Reference to Related Inventions

This invention is related to those inventions described in US patent application 12,842,327, titled "Jaw Movement Mechanism and Method for Surgical Tool", and US patent application 12/842,399 titled "Surgical Tool and Method Using Crossbar Lever", and US patent application 12/842,659, titled "Tissue Fusion System and Method of Performing a Functional Verification Test", all of which are filed concurrently herewith and all of which are assigned to the assignee hereof. The subject matter of these applications is incorporated herein by this reference.

### Field of the Invention

This invention relates to a thermal tissue operating system, which is also referred to generically as a tissue fusion system. More particularly, the present invention relates to a new and improved self-test in which the energy generating capability of an energy source of such a system is tested independently of a handpiece which normally delivers the thermal energy to the tissue, as well as verifying that other functional features of the system are properly operational prior to use of the system in a surgical procedure.

### Background

A thermal tissue operation involves simultaneously compressing and heating tissue to seal together pieces of tissue, to cut a single piece of tissue into separate parts, or to sequentially seal pieces of tissue and then cut the sealed tissue. Tissue cutting occurs in the same manner as tissue sealing, except that additional energy and heat are applied to the tissue to cause it to sever. Typical thermal tissue operations involve sealing blood vessels during surgery to prevent bleeding and blood loss. Sealing a blood vessel before severing it between spaced apart sealed locations or in the middle of single sealed location completely avoids blood loss.

A thermal tissue operating system includes a handpiece which is connected to an energy source. The handpiece has a pair of opposing jaws between which the tissue is mechanically compressed. Electrical power from the energy source is converted to thermal heat energy in at least one of the opposing jaws, and the heat is conducted into the compressed tissue. The characteristics of the electrical energy applied to the jaws control the characteristics of the heat energy conducted into the jaws. The characteristics of the thermal energy transferred to the tissue and the time during which the thermal energy is transferred constitute an individual thermal tissue operation, i.e. a tissue sealing operation, a tissue cutting operation, or a combined tissue cutting and sealing operation. Usually, the entire surgical procedure is completed by performing many separate individual thermal tissue operations.

A thermal tissue operating system can be subject to a number of external influences, such as accidental mishandling and improper use, for example. Such external influences have the potential to adversely affect the proper operation of the system. A malfunctioning or improperly functioning system may inadequately seal tissue, inadequately cut tissue, inadequately seal and cut the tissue, and otherwise complicate the surgical procedure.

If the thermal tissue operating system is functioning adversely, and the adverse functionality is discovered during the surgical procedure, the procedure must be delayed while the faulty component is replaced. Delaying the surgical procedure increases the trauma to the patient. However, the adverse functionality may not manifest itself during the procedure. Instead, the adverse functionality may only become apparent after the procedure has been completed. Leaking tissue seals which occur or are discovered after the procedure has been completed require a second surgical procedure to be performed to stop internal fluid leakage. Such a second surgical procedure is a source of substantial additional trauma to the patient.

### Summary of the Invention

It is desirable to identify potential problems with a thermal tissue operating system before it is used in a surgical procedure. An early identification of any problem avoids subsequent surgical complications and reduces the trauma on the patient caused by prolonging the initial surgical procedure or by performing subsequent surgical procedure to correct inadequate thermal tissue operations performed during the prior procedure.

The present invention is useful to quickly and reliably identify a power delivery problem of an energy source of a thermal tissue operating system. Energy delivery problems are identified by tests performed before the system is used in a surgical procedure. Preferably, the tests are performed automatically as part of a startup procedure such as a power on self test (POST). Current and voltage measurements of electrical energy delivered to a handpiece and/or to a substitute internal, load-simulation heating element within the energy source are used to identify potential problems with the power delivery capability of the energy source.

One aspect of the invention involves testing the functional interaction of the energy source of the thermal tissue operating system with a handpiece that connects to the energy source and with an alternatively connectable internal, load-simulation heating element within the energy source. The handpiece includes a pair of opposing jaws which contact and compress tissue during the thermal tissue operation. At least one of the jaws includes a jaw heating element for converting electrical power into thermal heat energy to be applied in the tissue. The energy source creates a heater power signal having voltage and current. The heater power signal is applied to a jaw heating element during the thermal tissue operation. The energy source further includes an internal load-simulation heating element, and a controllable switch directs the heater power signal to the jaw heating element or the simulation heating element. In a deactivated state, the controllable switch directs the heater power signal to the simulation heating element, and in an activated state, the controllable switch directs the heater power signal to the jaw heating element. A controller of the energy source controls the controllable switch to assume the activated and deactivated states. A sensor senses one or both of the voltage and/or current of the heater power signal and supplies a related sense signal to the controller. The controller receives the sense signal, controls the controllable switch into the activated and deactivated states and determines whether the controllable switch correctly occupies each intended state. In this manner, aspects of proper functionality of the energy source are determined, without connecting a handpiece to the energy source.

Another aspect of the invention involves a method of performing a test of a thermal tissue operating system. The method comprises establishing one state of a controllable switch in which electrical power should be conducted to a simulation heating element, conducting electrical power through the controllable switch when the controllable switch is in the one state, measuring one or both of the voltage and/or current of the electrical power conducted through the simulation heating element to obtain a measured value, referencing a predetermined range of expected values of the measured value indicative of normal voltage or current conducted through the simulation heating element, and communicating an error message when the measured value is not within the predetermined range of expected values.

Subsidiary features of the invention relate to some or all of the following: performing the same test on each of two jaw heating elements of the handpiece in which there is a controllable switch, a simulation heating element and a sensor associated with each jaw heating element; conducting power to the first and second simulation heating elements individually and separately while determining whether the first and second controllable switches correctly occupy their activated and deactivated states; using a control processor and a monitor processor functioning jointly as the controller to independently determine whether the first and second controllable switches each correctly occupy their activated and deactivated states; and using at least one peak detector as a sensor which is operative to detect and hold a value corresponding to a maximum value of at least one of the current or voltage of the signal sensed during the sample time interval, detecting the value at a relatively early point in the sample time interval and detecting the value again at a relatively late point in the sample time interval, and comparing the relatively early and late values, and indicating an insufficiently operative peak detector by a predetermined difference between the values at the relatively early and late points in the sample time interval and comparing that difference to a predetermined range of acceptable differences. Other subsidiary features are described below.

A more complete appreciation of the present invention and its scope may be obtained from the accompanying drawings, which are briefly summarized below, from the following detailed description of a presently preferred embodiment of the invention, and from the appended claims.

### Brief Description of the Drawings

Fig. 1 is a perspective view of a handpiece and an energy source of a thermal tissue operating system which incorporates the present invention.
Figs. 2A and 2B are graphs showing temperature versus time profiles for two different tissue sealing operations performed by the use of the thermal tissue operating system shown in Fig. 1.
Fig. 3 is a graph showing a temperature versus time profile for a tissue cutting operation performed by the use of the thermal tissue operating system shown in Fig. 1.
Fig. 4 is a graph showing a temperature versus time profile for a combined tissue sealing and cutting operation performed by use of the thermal tissue operating system shown in Fig. 1.
Fig. 5 is a block diagram of certain electrical components of the energy source and the handpiece shown in Fig. 1.
Fig. 6 is a more detailed block and schematic diagram of the energy source and handpiece shown in Fig. 5.

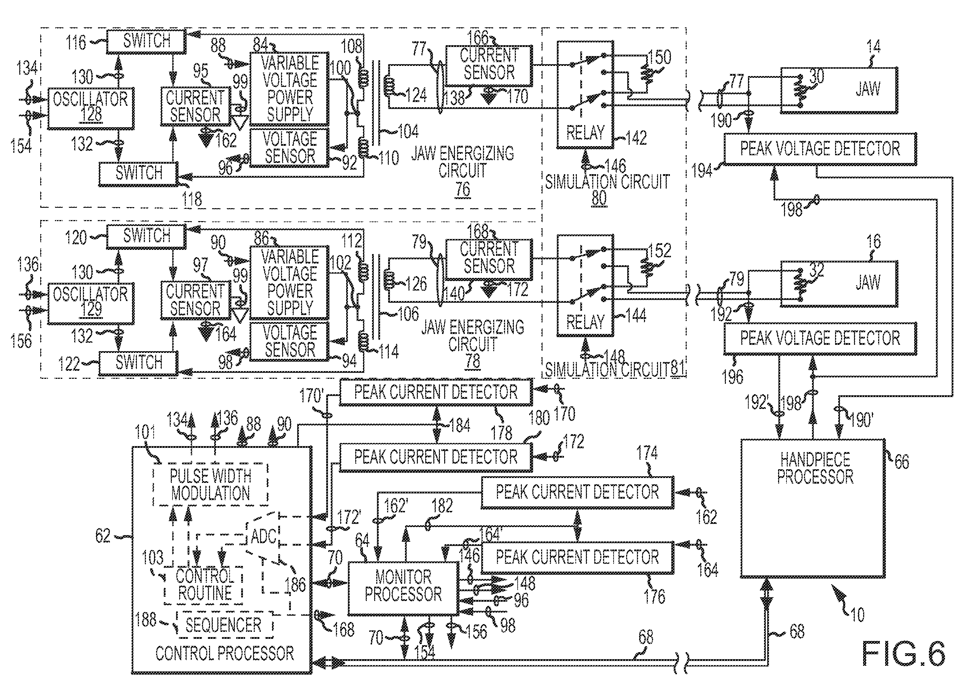

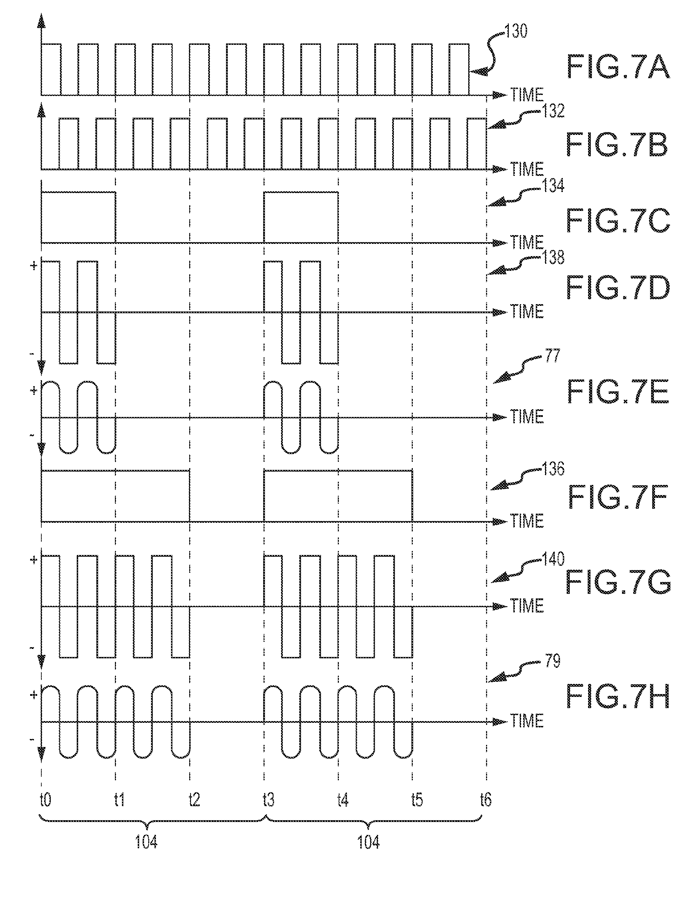

Figs. 7A-7H are graphs of exemplary signals in the energy source shown in Fig. 6, all of which share a common time axis. Specifically for two sequential control cycles, Figs. 7A and 7B show opposite phase square wave signals generated by an oscillator of one jaw energizing circuit of the energy source; Fig. 7C shows a relatively low duty cycle gate control signal supplied by a controller to an oscillator of one jaw energizing circuit of the energy source; Fig. 7D shows an input power signal to a transformer of the jaw energizing circuit, formed in response to the gate control signal shown in Fig. 7C; Fig. 7E shows a heater power signal created by the transformer of the jaw energizing circuit in response to the input power signal shown in Fig. 7D; Fig. 7F shows a relatively high duty cycle gate control signal supplied by the controller to an oscillator of one jaw energizing circuit of the energy source; Fig. 7G shows an input power signal to a transformer of the jaw energizing circuit, formed in response to the gate control signal shown in Fig. 7F; and Fig. 7H shows a heater power signal created by the transformer of the jaw energizing circuit in response to the input power signal shown in Fig. 7G.
Figs. 8A-8C are graphs of signals exemplary of those present in the energy source and handpiece shown in Fig. 6, all of which share a common time axis. Specifically, Fig. 8A shows a waveform illustrative of either a voltage or current sense signal applied to a peak detector; Fig. 8B shows a reset signal supplied to the peak detector; and Fig. 8C shows a peak signal representative of the peak value which is detected and held by the peak detector in response to the sense signal shown in Fig. 8A, with the sense signal also shown in phantom in Fig. 8C.
Fig. 9 is a graph showing an exemplary characteristic relationship of temperature versus resistance of a jaw heating element of the handpiece shown in Figs. 5 and 6.
Fig. 10 is a flow chart showing a relay test performed by the energy source shown in Figs. 5 and 6.
Fig. 11 is a flow chart showing a power test performed by the energy source shown in Figs. 5 and 6.
Fig. 12 is a flow chart showing a peak detector test performed by the energy source and handpiece shown in Figs. 5 and 6.
Fig. 13 is a flow chart showing a combined power on self test of the energy source and handpiece shown in Figs. 5 and 6.

### Detailed Description

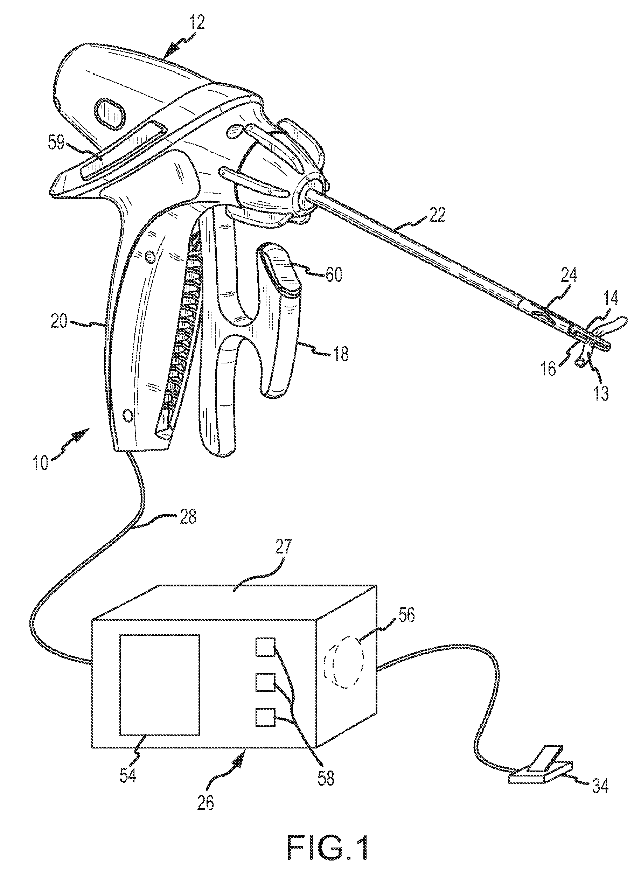

A thermal tissue operating system 10 in which the present invention is incorporated is shown in Fig. 1. The system 10 includes a handpiece 12 which is manipulated by a surgeon to grasp and compress tissue (exemplified by a vessel 13) between jaws 14 and 16 of the handpiece 12, and to simultaneously apply thermal heat energy from the jaws 14 and 16 to the compressed tissue in a thermal tissue operation. The thermal tissue operation may seal multiple pieces of the tissue together, cut a single piece of tissue into separate parts, or sequentially seal and then cut tissue.

The jaws 14 and 16 are brought together to compress the tissue by squeezing a lever 18 toward an adjacent handgrip 20 of the handpiece 12. Internal mechanical components of the handpiece 12 (not shown but described in the above-application US/SN 12/842,399) convert the pivoting movement of the lever 18 relative to the handgrip 20 into motion which is transferred through a shaft 22 to a jaw movement mechanism 24 (which is described in detail in the above application number US/SN 12/842,327). The jaw movement mechanism 24 converts the longitudinal movement from the shaft 22 into movement to move the jaws 14 and 16 toward and away from one another. Movement of the jaws 14 and 16 toward one another grips and compresses the tissue between the jaws. Movement of the jaws 14 and 16 away from one another opens the jaws sufficiently to accept tissue between them before gripping and compressing the tissue and releases any tissue previously gripped.

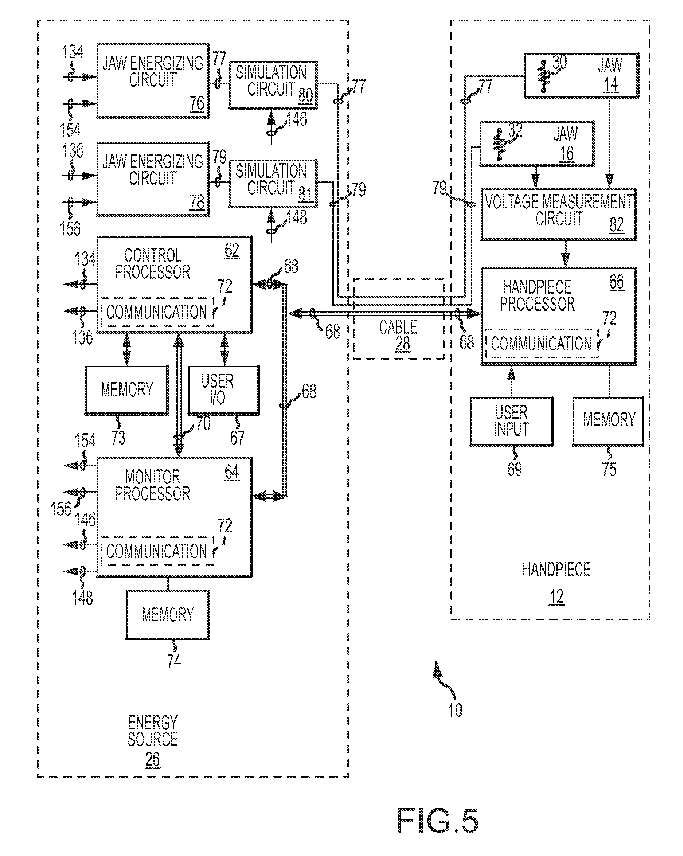

The thermal tissue operating system 10 also includes an electrical energy source 26 which is connected by a cable 28 to the handpiece 12. The energy source 26 includes electrical components (Figs. 5 and 6) housed within an enclosure 27. The energy source 26 supplies electrical power through the cable 28 to a pair of heat-producing resistive elements (30 and 32, Figs. 5 and 6) that are embedded within or associated with the jaws 14 and 16 (Fig. 1). Electrical power conducted through the jaw heating elements (30 and 32, Figs. 5 and 6) is converted into heat energy and is applied to the tissue gripped and compressed between the jaws 14 and 16 during the thermal tissue operation.

Electrical power is supplied when the lever 18 is pulled into proximity with the handgrip 20 and one of the switches 59 or 60 is pressed, thereby delivering a user activation signal from the handpiece 12 to the energy source 26. In response to the user activation signal, the energy source 26 delivers electrical power to the jaw heating elements (30 and 32, Figs. 5 and 6) of the jaws 14 and 16. Alternatively, the activation signal may be supplied by pulling the lever 18 into proximity with the handgrip 20 and pressing a foot switch 34 which is connected to the energy source 26. The surgeon depresses the foot switch 34 with his or her foot.

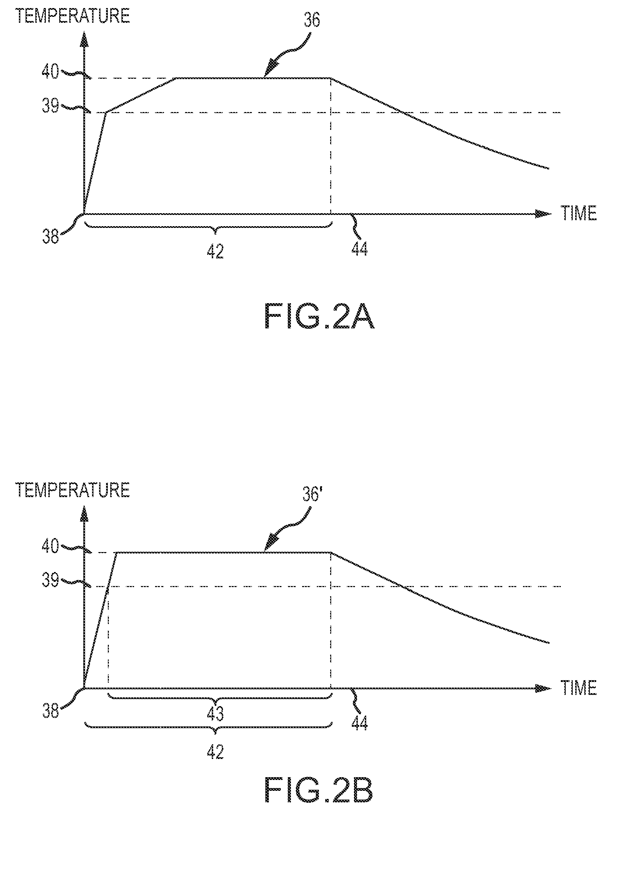

To accomplish a thermal tissue operation, the energy source 26 delivers electrical power to the jaw heating elements (30 and 32, Figs. 5 and 6), and that electrical power is converted into thermal energy and applied to the tissue. The thermal energy is delivered to the tissue compressed between the jaws 14 and 16 in accordance with a temperature versus time profile (36 or 36', 37, 46, Figs. 2A or 2B, 3 and 4) which is established for each type of thermal tissue operation. The temperature is achieved and controlled by the rate of energy delivered from the energy source 26 using temperature-based feedback signals from the jaws 14 and 16 of the handpiece 12. The energy source 26 controls the rate of electrical energy delivery to the jaw heating elements based on the measurement of the temperature at the jaws 14 and 16 for the duration of the thermal tissue operation. Desired temperature versus time profiles to accomplish the thermal tissue operations are shown in Figs. 2A, 2B, 3 and 4.

One exemplary temperature versus time profile 36 for accomplishing a tissue sealing operation is shown in Fig. 2A. At time 38, the energy source 26 receives the activation signal to initiate the tissue sealing operation. The energy source 26 immediately delivers relatively high or maximum power to the jaw heating elements (30 and 32, Figs. 5 and 6) to rapidly achieve a preliminary sealing temperature 39. Thereafter, the energy source 26 delivers a relatively lower amount of power to the jaw heating elements to achieve the final sealing temperature 40 less rapidly. Reducing the rate of temperature increase from the preliminary sealing temperature 39 to the final sealing temperature 40 reduces the possibility of an overshoot in the final sealing temperature 40. Upon reaching the final sealing temperature 40, the energy source 26 regulates the amount of electrical power supplied to the jaw heating elements to maintain the temperature 40 over the remaining portion of a tissue sealing time interval 42.

The length of tissue sealing time interval 42 ends when either a predetermined minimum amount of electrical energy has been transferred to the jaw heating elements and a predetermined minimum amount of time has elapsed from the activation time 38, or a predetermined maximum amount of time for the sealing time interval 42 has elapsed. The amount of electrical energy transmitted to the tissue is the sum of the electrical energy transmitted to both jaw heating elements (30 and 32, Figs. 5 and 6) of the jaws 14 and 16 (Fig. 1). The total amount of electrical energy delivered throughout the progression of the time interval 42 is calculated and compared to the predetermined combined minimum amount of electrical energy, and the time elapsed since the start of the tissue sealing operation at 38 is compared with the predetermined minimum and maximum times for the tissue sealing operation to determine when either of the two above-described conditions for ending the tissue sealing operation are met.

When either of the two above-described conditions for ending the tissue seal operation are met, the energy source 26 terminates the delivery of power to the jaw heating elements, allowing the jaw heating elements to cool and decrease in temperature. The preferred sealing temperature 40 is approximately 170°C, and the predetermined minimum and maximum tissue sealing times vary from approximately 2 to 5 seconds, respectively. Preferably, the sealing temperature 40, the minimum and maximum tissue sealing times, and other information are stored within a handpiece processor 66 (Figs. 5 and 6) of the handpiece 12 and are downloaded to the power system 26 prior to performing a thermal tissue operation. Different values of the thermal tissue operation-related variables are stored in different handpieces having different jaw heating elements with different electrical and thermal characteristics, to perform thermal tissue operations with the different types of handpieces.

Another exemplary temperature versus time profile 36' for accomplishing a tissue sealing operation is shown in Fig. 2B. The temperature versus time profile 36' is similar to that profile 36 shown in Fig. 2A, except that the energy source 26 delivers relatively high or maximum power to the jaw heating elements (30 and 32, Figs. 5 and 6) to achieve the final sealing temperature 40 more rapidly. Upon reaching the final sealing temperature 40, the energy source 26 regulates the amount of electrical power supplied to the jaw heating elements to maintain the temperature 40 during a final temperature maintenance time interval 43 after the final sealing temperature 40 is initially reached. The entire tissue sealing time interval 42 is therefore slightly greater in time than the final temperature maintenance interval 43, because the entire tissue sealing time interval 42 also includes the time between the assertion of the initial user activation signal at 38 until the final sealing temperature 40 is reached at the beginning of the final temperature maintenance interval 43.

In the tissue sealing temperature versus time profile 36', the final sealing temperature 40 is maintained for the duration of the maintenance time interval 43. The tissue sealing time interval 42 ends when the final sealing temperature 40 has been maintained within slight limits of variation for the duration maintenance time interval 43. No determination is made of whether a predetermined minimum amount of electrical energy has been transferred to the jaw heating elements when the tissue sealing profile 36' is performed. The time elapsed since the activation time 38 is measured, and if that time exceeds a predetermined maximum amount of time, the thermal tissue sealing operation is terminated because under the assumption that some issue has arisen which will prevent the proper execution of a sealing thermal tissue operation.

In the tissue sealing thermal operation represented by the temperature versus time profile 36', the final temperature maintenance interval 43 is approximately 2 seconds in time duration and the final sealing temperature 40 is approximately 150°C. Timing the 2 second final temperature maintenance interval 43 begins when the temperature is within approximately 10°C of the desired 150°C final sealing temperature 40. The temperature 39 exemplifies the starting point for measuring the temperature maintenance interval 43, because the temperature 39 is approximately 10°C less than the final desired sealing temperature. The benefit of the tissue sealing profile 36' over the tissue sealing profile 36 (Fig. 2A) is that, in some cases involving some tissues in some procedures, adequate tissue seals may be obtained using a lower temperature for a shorter duration of time.

The predetermined maximum time duration allowable for a thermal tissue sealing operation, the final desired 150°C temperature, and other information are stored within a handpiece processor 66 (Figs. 5 and 6) of the handpiece 12 and are downloaded to the energy source 26 prior to performing a thermal tissue operation. Different values of the thermal tissue operation-related variables are stored in different handpieces having different jaw heating elements with different electrical and thermal characteristics, to perform thermal tissue operations with the different types of handpieces.

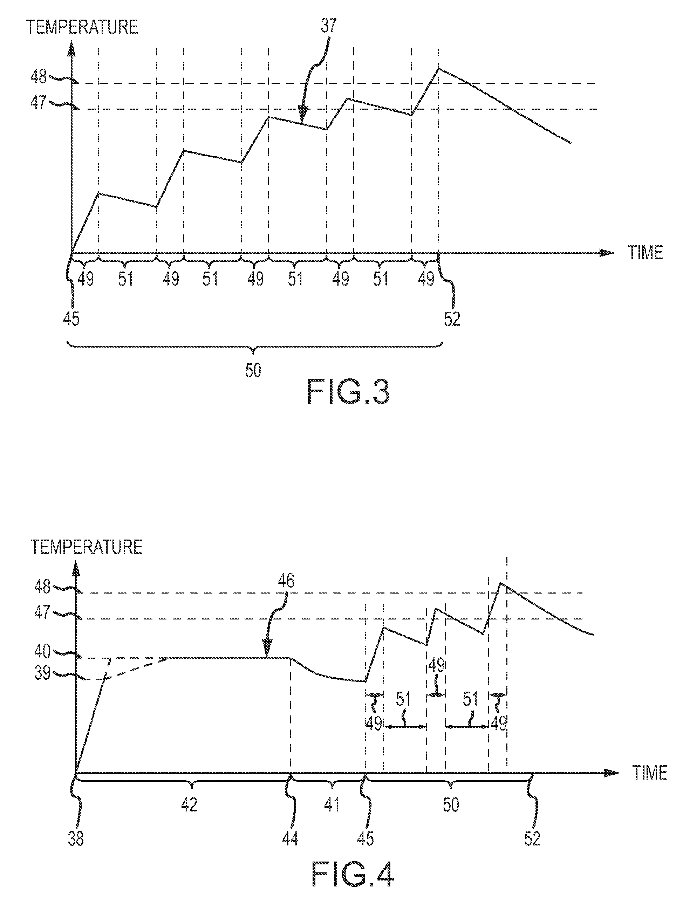

A tissue cutting operation can also be performed independently of a tissue seal operation. A tissue cutting operation is typically performed after one or more tissue sealing operations have sealed the tissue or vessel which is to be cut. An exemplary temperature versus time profile 37 for accomplishing a tissue cut operation is shown in Fig. 3. At time 45, an activation signal is delivered to the energy source 26, and the tissue cutting operation starts. During the tissue cutting operation, the energy source 26 alternately supplies relatively high power to the jaw heating elements during power delivery periods 49 followed by terminating the supply of power to the jaw heating elements during power off periods 51. The power delivery periods 49 are preferably about 100ms in time duration and the power off periods 51 are preferably about 200ms in duration. The power delivery periods 49 and power off periods 51 are repeated in succession until the temperature of the jaw heating elements reaches a preliminary cutting temperature 47. Thereafter, a lower amount of power is delivered during the following power delivery periods 49. The power delivery periods 49 and power off periods 51 are continued until the temperature of the jaw heating elements reaches a final cutting temperature 48, at which time 52 the tissue cutting operation is complete and the supply of power to the jaw heating elements is terminated completely.

Preferred temperatures for the respective preliminary and final cutting temperatures 47 and 48 vary depending on the electrical and thermal characteristics of the jaw heating elements, but are generally between 200-240°C and 270-280°C, respectively. A slight amount of overshoot of both the preliminary and final cutting temperatures 47 and 48 may occur during the respective power delivery periods 49 when the temperatures 47 and 48 are first reached. This slight overshoot is due to the energy source 26 completing the delivery of power during the power delivery period 49 when the temperatures 47 and 48 are first attained.

The time between the start time 45 and finish time 52 of the tissue cutting operation is the cutting time interval 50. The cutting time interval 50 varies for different tissue cutting operations due to differences in the amount of tissue to be cut between the jaws 14 and 16 (Fig. 1), the temperature of the jaw heating elements at the start time 45 of the cutting time interval 50, and the electrical and thermal characteristics of the jaw heating elements, among other factors.

The amount of energy delivered during the cutting time interval 50 is sufficient to disintegrate the tissue squeezed and compressed between the jaws 14 and 16 (Fig. 1). The disintegration permits the tissue to be separated into parts, without destroying, disintegrating or otherwise adversely compromising the quality of a seal which may be closely located on opposite sides of a generally linear delineation where the tissue cutting or disintegration occurs.

The successive power delivery periods 49 and power off periods 51 cause the temperature versus time profile 37 for the tissue cutting operation to resemble an inclined saw tooth shape. The inclined saw tooth shaped tissue cutting profile has been discovered to possess superior tissue cutting characteristics versus a conventional ramp profile when the temperature is continually increased until a desired final cutting temperature is reached.

The temperature versus time profiles 36 (Fig. 2A) and 37 (Fig. 3) can be combined to form a temperature versus time profile 46, shown in Fig. 4, for a combined tissue sealing and cutting operation. The temperature versus time profiles 36' (Fig. 2B) and 37 (Fig. 3) can also be combined to form a temperature versus time profile (not specifically shown but similar to the profile 46 shown in Fig. 4) for a combined tissue sealing and cutting operation. The combined tissue sealing and cutting temperature versus time profile 46 resembles the temperature versus time profile 36 (Fig. 2A) or 36' (Fig. 2B) from a starting time 38 to an intermediate time 44 when the tissue sealing profile portion (36 or 36', Figs. 2A or 2B) of the operation is complete. The tissue is then allowed to cool slightly during a cooling time interval 41 between the end of the tissue sealing operation at time 44 and the start of the tissue cutting operation at time 45. The cooling time interval 41 is approximately one second in duration, and is instrumental in contributing to a more effective and efficient tissue sealing and cutting operation, compared to performing the tissue sealing and cutting operations directly in sequence without a cooling time interval 41.

Between times 45 and 52, the temperature versus time profile 46 resembles the temperature versus time profile 37 (Fig. 3) of the tissue cutting operation. The energy source 26 alternately supplies relatively high power to the jaw heating elements during power delivery periods 49 followed by terminating the supply of power to the jaw heating elements during power off periods 51. The power delivery periods 49 are preferably about 100ms in time duration and the power off periods 51 are preferably about 200ms in duration. The power delivery periods 49 and power off periods 51 are repeated in succession until the temperature of the jaw heating elements reaches a preliminary cutting temperature 47. Thereafter, a lower amount of power is delivered during the following power delivery periods 49. The power delivery periods 49 and power off periods 51 are continued until the temperature of the jaw heating elements reaches a final cutting temperature 48, at which time 52 the tissue cutting operation is complete and the supply of power to the jaw heating elements is terminated completely.

Preferred temperatures for the respective preliminary and final cutting temperatures 47 and 48 vary depending on the electrical and thermal characteristics of the jaw heating elements, but are generally between 200-240°C and 270-280°C, respectively. A slight amount of overshoot of both the preliminary and final cutting temperatures 47 and 48 may occur during the respective power delivery periods 49 when the temperatures 47 and 48 are first reached. This slight overshoot is due to the energy source 26 completing the delivery of power during the power delivery period 49 when the temperatures 47 and 48 are first attained.

The time between the start time 45 and the finish time 52 of the tissue cutting operation is the cutting time interval 50. The cutting time interval 50 varies for different tissue cutting operations due to differences in the amount of tissue to be cut between the jaws 14 and 16 (Fig. 1), the temperature of the jaw heating elements at the start time 45 of the cutting time interval 50, and the electrical and thermal characteristics of the jaw heating elements, among other factors.

As shown in Fig. 1, a display 54 and a speaker 56 are included within the enclosure 27 of the energy source 26. The display 54 and the speaker 56 convey information about the functional response characteristics of the thermal tissue operating system 10, during use of the system. The energy source 26 also includes mode selection controls or switches 58. The handpiece 12 includes selection thumb switches 59 on opposite sides of the handgrip 20 (only one selection switch 59 is shown in Fig. 1). The handpiece 12 also includes a finger selection switch 60 on the lever 18. The mode control switches 58 are used to select between a manual mode of operation and an automatic mode of operation. In the manual mode of operation, a tissue cut operation is activated by pulling the lever 18 back toward the handgrip 20 and then depressing one of the thumb switches 59. In the manual mode of operation, a tissue seal operation is activated by depressing the finger switch 60 when the lever 18 is pulled back toward the handgrip 20. In the automatic mode of operation, a combined tissue sealing and cutting operation is activated by depressing the switch 60 when the lever 18 is pulled back toward the handgrip 20. In the automatic mode of operation, pressing the switch 59 with the lever 18 pulled back toward the handgrip 20 activates a manual cut operation.

The present invention involves performing certain functional integrity tests of the energy source 26, separately and when the handpiece 12 is disconnected from the energy source 26, and/or when the handpiece 12 is connected to the energy source 26. The tests are preferably performed as part of a combined power on self test (POST) of the thermal tissue operating system 10. These self-tests ensure that the thermal tissue operating system is working properly to perform the tissue sealing, tissue cutting and combined tissue sealing and cutting operations. The details of these self-tests and the thermal tissue operating system 10 are described below in connection with Figs. 5-13.

As shown in Fig. 5, the energy source 26 includes a control processor 62 and a monitor processor 64. The control processor 62 generally controls the operation and overall functionality of the energy source 26, as well as performing and participating in the performance of the self-tests described herein. The monitor processor 64 monitors the operation of the control processor 62 and otherwise performs many of its own functional tests to ensure that the control processor 62 and other sub-components are operating as expected.

A handpiece processor 66 of the handpiece 12 controls the operation of the handpiece 12, in response to signals from the lever 18 and switches 59 and 60 (Fig. 1) and signals from the control processor 62 communicated over a communication bus 68 which is part of the cable 28 (Fig. 1) connecting the energy source 26 with the handpiece 12. The monitor processor 64 is also connected to the communication bus 68 to enable it to communicate with the handpiece processor 66 and the control processor 62. In addition, the control processor 62 and the monitor processor 64 are directly connected together by a separate bus 70, for direct communication of signals between those processors 62 and 64.

Either individually or by cooperative combination of functionalities with one or more of the other processors, one or more of the processors 62, 64 and 66 constitute a controller for the energy source 26, a controller for the handpiece 12, and a controller for the thermal tissue operating system 10. Even though the components 62, 64 and 66 are described in their exemplary form as processors, any type of computational device, data processing device, controller or programmable logic gate device, which is capable of performing the functions described herein as attributable to the components 62, 64 and 66, may constitute processors 62, 64 and 66.

Communication between the processors 62, 64 and 66 is accomplished by using a predefined communication protocol, which is implemented within a communication routine 72 of the control processor 62, the monitor processor 64 and the handpiece processor 66. Executing the communication routine 72 allows the transfer of information between the processors 62, 64 and 66 over the bus 68. The processors 62, 64 and 66 include memory modules 73, 74 and 75, which store the programs that the processors 62, 64 and 66 execute to achieve their respective functionalities. In addition, user input and output (I/O) 67 is communicated to the control processor 62 by use of the display 54, the speaker 56 and the front panel controls 58 of the energy source (Fig. 1). User input 69 is also communicated to the handpiece processor 66 by movement of the lever 18 and the depression of the thumb switches 59 and finger switch 60 (Fig. 1).

The energy source 26 also includes a first jaw energizing circuit 76 which supplies a heater power signal 77 to the heating element 30 in the jaw 14 of the handpiece 12. The energy source 26 also includes a second jaw energizing circuit 78 which supplies a heater power signal 79 to the heating element 32 in the jaw 16 of the handpiece 12. The heater power signals 77 and 79 establish the amount of electrical power delivered to the jaw heating elements 30 and 32. The heater power signals 77 and 79 are converted into thermal energy by the jaw heating elements 30 and 32 to accomplish the thermal tissue operations. The heater power signals 77 and 79 are conducted from the energy source 26 to the handpiece 12 through conductors in the cable 28.

The jaw energizing circuits 76 and 78 are independently and respectively controlled by the control processor 62 asserting gate control signals 134 and 136. The gate control signals 134 and 136 control characteristics of the separate heater power signals 77 and 79 delivered to each jaw heating element 30 and 32, thereby allowing the temperature of each jaw heating element 30 and 32 to be individually controlled in response to individual temperature feedback controls from each jaw heating element. Independent regulation of the temperature of each heating element 30 and 32 allows the temperature of the tissue gripped between the jaws 14 and 16 to be more precisely controlled to achieve the desired temperature characteristics for a seal operation, a cut operation and a combined seal and cut operation. The monitor processor 64 enables the jaw energizing circuits 76 and 78 to deliver the heater power signals 77 and 79 by asserting enable signals 154 and 156, respectively. Whenever an enable signal 154 or 156 is de-asserted, the respective jaw energizing circuit 76 or 78 will not create the heater power signal 77 or 79.

Simulation circuits 80 and 81 are connected to the jaw energizing circuits 76 and 78 to receive the heater power signals 77 and 79, respectively, under the control of the monitor processor 64, when it is desired to conduct certain functional integrity tests described below. When deactivated by the monitor processor 64 deasserting activation signals 146 and 148, the simulation circuits 80 and 81 conduct the heater power signals 77 and 79 through internal load simulating heating elements (150 and 152, Fig. 6) within the simulation circuits 80 and 81, respectively. When activated by the monitor processor 64 asserting the activation signals 146 and 148, the simulation circuits 80 and 81 conduct the heater power signals 77 and 79 to the heating elements 30 and 32 of the jaws 14 and 16, respectively. Conducting the functional integrity tests of the energy source 26 with the simulation circuits 80 and 81 ensures that the thermal tissue operating system is working properly.

The handpiece 12 includes a voltage measurement circuit 82 that detects the voltage across the heating elements 30 and 32 of the jaws 14 and 16 when the heater power signals 77 and 79 cause current flow through those heating elements 30 and 32. The handpiece processor 66 communicates the voltage values from the measurement circuit 82 over the bus 68 to the control and monitor processors 62 and 64. The control processor 62 uses those voltage values to calculate power and energy delivered to and consumed by the heating elements 30 and 32. Measuring the voltage across the heating elements 30 and 32 at the jaws provides greater accuracy in the measurement of the power and energy consumed by the jaw heating elements 30 and 32, because losses resulting from conducting the power heating signals 77 and 79 through the conductors of the cable 28 are not involved in the voltage values detected by the measurement circuit 82. Independent determinations of the power and energy delivered to and consumed by each of the heating elements 30 and 32 facilitate individual control over each of the heating elements 30 and 32.

More details concerning the jaw energizing circuits and 76 and 78, the simulation circuits 80 and 81 and the functionality of the control and monitor processors 62 and 64 of the energy source 26, as well as the heating elements 30 and 32, the measurement circuit 82 and the handpiece processor 66 of the handpiece 12, are shown and discussed in connection with Fig. 6.

The jaw energizing circuits 76 and 78 are each substantially identical in construction and functionality, although each jaw energizing circuit 76 and 78 is separately controllable. Each jaw energizing circuit 76 and 78 respectively includes a variable voltage power supply 84 and 86. Each variable voltage power supply 84 and 86 is connected to a conventional commercial energy source (not shown). Each power supply 84 and 86 converts commercial power to direct current power at a voltage established by each power supply 84 and 86 in response to voltage control signals 88 and 90 supplied by the control processor 62 to each power supply 84 and 86, respectively. Each jaw energizing circuit 76 and 78 is therefore capable of supplying the heater power signal 77 and 79, respectively, at different and individually controlled voltage levels established by the control signals 88 and 90.

Voltage sensors 92 and 94 are connected to sense the output voltage from the variable voltage power supplies 84 and 86. The voltage sensors 92 and 94 supply voltage sense signals 96 and 98 to the monitor processor 64 in response to the voltages of the electrical energy delivered from the variable voltage power supplies 84 and 86. The ability to individually adjust the voltage from each power supply 84 and 86 allows adjustment to compensate for slight variations in the resistances of each jaw heating element 30 and 32. Changing the voltage to compensate for a slightly changed resistance of a jaw heating element 30 or 32 causes each jaw heating element to consume approximately the same amount of electrical energy and thereby generate approximately the same amount of thermal energy, for similar gate control signals applied, as discussed below.

Electrical energy at the output voltage of the power supplies 84 and 86 is supplied to center taps 100 and 102 of a center tapped primary winding of power output transformers 104 and 106, respectively. The primary windings of the power output transformers 104 and 106 are therefore divided into two winding segments 108, 110 and 112, 114 by the center taps 100 and 102, respectively. The upper (as shown) winding segments 108 and 112 are connected to switches 116 and 120, respectively. The lower (as shown) winding segments 110 and 114 are connected to switches 118 and 122, respectively. When the switches 116 and 120 are conductive, current is conducted through the winding segments 108 and 112 from the variable voltage power supplies 84 and 86 through current sensors 95 and 97, respectively, to reference potential 99. When the switches 118 and 122 are conductive, current is conducted through the winding segments 110 and 114 from the variable voltage power supplies 84 and 86, through the current sensors 95 and 97, respectively, to the reference potential 99.

Each of the jaw energizing circuits 76 and 78 includes its own oscillator 128 and 129, respectively. The switches 116 and 118 conduct in response to signals generated by the oscillator 128, and the switches 120 and 122 conduct in response to signals generated by the oscillator 129. The oscillators 128 and 129 each generate two substantially similar or identical relatively high frequency, e.g. 50 kHz, square wave signals 130 and 132 (Figs. 7A and 7B). The square wave signals 130 and 132 are phase shifted with respect to one another by 180 degrees. The square wave signal 130 is applied to the switches 116 and 120. The square wave signal 132 is applied to the switches 118 and 122. The switches 116-122 are capable of conducting current from the primary winding segments 108-114 of the of the power output transformers 104 and 106, only when the square wave signals 130 and 132 are a positive value. During the times that the square wave signals 130 and 132 are at reference or zero value, the switches 116-122 are not capable of conducting.

A gate control signal 134 is applied from the control processor 62 to the oscillator 128, and a gate control signal 136 is applied from the control processor 62 to the oscillator 129. Upon assertion of the gate control signal 134, the oscillator 128 conducts the square wave signals 130 and 132, respectively, for the duration of the assertion of the gate control signal 134. Because the square wave signals 130 and 132 are phase shifted with respect to one another by 180 degrees, the alternating conductivity of the switches 116 and 118 conducts current in opposite directions through the primary windings 108 and 110 from the center tap 100, thereby establishing a primary alternating current signal 138 (Fig. 7D) which is conducted through the primary winding segments 108 and 110 of the power output transformer 104. Similarly, upon assertion of the gate control signal 136, the oscillator 129 conducts the square wave signals 130 and 132, respectively, for the duration of the assertion of the gate control signal 136. Because the square wave signals 130 and 132 are phase shifted with respect to one another by 180 degrees, the alternating conductivity of the switches 120 and 122 conducts current in opposite directions through the primary windings 112 and 114 from the center tap 102, thereby establishing a primary alternating current signal 140 (Fig. 7G) which is conducted through the primary winding segments 112 and 114 of the power output transformer 106. The primary alternating current signals 138 and 140 induce the heater power signals 77 and 79 from the secondary windings 124 and 126 of the power output transformers 104 and 106, respectively.

The amount of electrical energy contained in the heater power signals 77 and 79 is directly related to the voltage from the variable voltage power supplies 84 and 86, respectively, and is also directly related to the time duration of the gate control signals 134 and 136. Asserting the gate control signals 134 and 136 for a longer time duration results in the switches 116, 118 and 120, 122 conducting the primary alternating current signals 138 and 140 through the primary winding segments 108, 110 and 112, 114 of the power output transformers 104 and 106 for a greater duration of time, thereby causing greater energy content in the heater power signals 77 and 79, respectively. Conversely, asserting the gate control signals 134 and 136 for a shorter time duration results in the switches 116, 118 and 120, 122 conducting the primary alternating current signals 138 and 140 through the primary winding segments 108, 110 and 112, 114 of the power output transformers 104 and 106 for lesser duration of time, thereby causing lesser energy in the heater power control signals 77 and 79.

The control processor 62 independently controls the duration of the gate control signals 134 and 136, thereby controlling the amount of electrical energy delivered to the jaw heating elements 30 and 32 for conversion into thermal energy to establish and maintain the desired temperature of the jaw heating elements. The thermal loads experienced by each of the jaws 14 and 16 are somewhat different. It is because of the different thermal loads that the control processor 62 exercises independent control over each of the jaw energizing circuits 76 and 78 by separately establishing the time duration of each of the gate control signals 134 and 136, which in turn separately establish the electrical energy content of the heater power signals 77 and 79. Figs. 7C and 7F illustrate the separate and individual control of each gate control signal 134 and 136.

The power and consequently temperature control of the jaw heating elements 30 and 32 is performed by the control processor 62 on a control cycle basis. A control routine 103 is executed by the control processor 62 in accordance with the selected thermal tissue operation, and the temperature versus time profile 36 or 36', 37 and 46 (Figs. 2A or 2B, 3 and 4, respectively) of the selected thermal tissue operation, in response to the user activation signal. The control routine 103 invokes a conventional feedback pulse width modulation routine 101 that establishes the time duration of the gate control signals 134 and 136 for each control cycle 104 in relation to the temperature of the jaw heating elements 30 and 32. The control processor 62 supplies the gate control signals 134 and 136 to the oscillators 128 and 129, and the duration of the gate control signals 134 and 136 establish the desired number of pulses of the square wave signals 130 and 132 conducted during each control cycle to create heater power signals 77 and 79.

The duty cycle of the gate control signals 134 and 136 during each control cycle 104 controls the amount of electrical energy delivered to the jaw heating elements during that control cycle, as understood by reference to Figs. 7A-7H. The exemplary signals shown in Figs. 7A-7H extend over two control cycles 104. The square wave signals 130 and 132 produced by the oscillators 128 and 129 are shown in Figs. 7A and 7B. A relatively low duty cycle gate control signal 134 supplied by the control processor 62 is shown in Fig. 7C. The relatively low duty cycle gate control signal 134 shown in Fig. 7C has an on time that extends from to to t₁ and an off time that extends from t₁ to t₃ in the first shown control cycle 104 and an on time that extends from t₃ to t₄ and an off time that extends from t₄ to t₆ in the second control cycle 104. The relatively low duty cycle of the gate control signal 134 creates the primary alternating current signal 138 shown in Fig. 7D that is formed by two cycles of square wave signals 130 and 132.

A relatively high duty cycle gate control signal 136 supplied by the control processor 62 is shown in Fig. 7F. The relatively high duty cycle gate control signal 136 shown in Fig. 7F has a much longer on time and a much shorter off time compared to the on and off times of the gate control signal 134 shown in Fig. 7C. The on time of the relatively high duty cycle gate control signal 136 shown in Fig. 7F extends from t₀ to t₂ and its off time extends from t₂ to t₃ in the first control cycle 104. Similarly in the second control cycle 104 shown in Fig. 7F, the longer on time extends from t₃ to t₅ and the shorter off time extends from t₅ to t₆. The relatively high duty cycle of the gate control signal 136 creates the primary alternating current signal 140 shown in Fig. 7G that is formed by four cycles of square wave signals 130 and 132.

Thus, the control processor 62 varies the amount of energy of the heater power signals 77 and 79 by varying the duty cycle of the gate control signals 134 and 136. Varying the duty cycle of the gate control signals 134 causes the oscillators 128 and 129 to vary the number of pulses of the square wave signals 130 and 132 conducted to the switches 116-122, which in turn varies the time duration that the primary alternating current signals 138 and 140 are present during each control cycle 104. Fewer and greater numbers of pulses of the square wave signals 130 and 132 during each control cycle 104 result in less and more electrical energy reaching the jaw heating elements 30 and 32 during each control cycle 104, respectively. The exemplary control cycles shown in Figs. 7A-7H have six pulses of square wave signals 130 and 132 forming each control cycle 104, for illustrative purposes only; in actuality, each control cycle 104 will typically have a considerably greater number of pulses of the square wave signals 130 and 132. In a practical embodiment of the thermal tissue operating system, the length of a control cycle 104 is about 5 ms.

The primary alternating current signals 138 and 140 are conducted through the primary winding segments 108, 110 and 112, 114 of output transformers 104 and 106, as shown in Fig. 6. In response, the transformers 104 and 106 respectively induce heater power signals 77 and 79 from their secondary windings 124 and 126. Other than slight reductions caused by the losses which occur in the transformers 104 and 106, the energy content of the heater power signals 77 and 79 is approximately the same as the energy content of the primary alternating current signals 138 and 140.

The heater power signals 77 and 79 are conducted to relays 142 and 144 of the simulation circuits 80 and 81, respectively. The relays 142 and 144 are activated and deactivated by the assertion and deassertion of relay activation signals 146 and 148 supplied by the monitor processor 64. When the relays 142 and 144 are deactivated, the heater power signal 77 and 79 pass through the relays 142 and 144 to load-simulation heating elements 150 and 152. The load-simulation heating elements 150 and 152 are a part of the energy source 26 and are located within the enclosure 27 (Fig. 1) of the energy source 26. When the relays 142 and 144 are activated, the heater power signals 77 and 79 are conducted through the cable 28 to the jaw heating elements 30 and 32 of the handpiece 12.

For the heater power signals 77 and 79 to reach the jaw heating elements 30 and 32 of the handpiece 12, the monitor processor 64 must be fully functional and must determine that the operation of the energy source 26 and handpiece 12 is appropriate and within safe limits. It is under these circumstances that the relay activation signals 146 and 148 are asserted by the monitor processor 64, to activate the relays 142 and 144 and thereby permit the heater power signals 77 and 79 to reach the jaw heating elements 30 and 32, respectively. The relays 142 and 144 are examples of controllable switches that receive control signals, such as the relay activation signals 146 and 148, to change between conductive states.

In addition to deactivating the relays 142 and 144 to terminate the supply of power to the jaw heating elements 30 and 32, the monitor processor 64 can separately terminate the creation of the heater power signals 77 and 79 in the jaw energizing circuits 76 and 78, by deasserting enable signals 154 and 156 applied to the oscillators 128 and 129, respectively. The oscillators 128 and 129 generate the square wave signals 130 and 132 only when the enable signals 154 and 156 are asserted by the monitor processor 64. When the enable signals 154 and 156 are de-asserted, the oscillators 128 and 129 do not generate the square wave signals 130 and 132, and the heater power signals 77 and 79 are not created.

When the switches 116, 118 and 120, 122 are conductive, the current flowing through those switches passes through current sensors 95 and 97. The current sensors 95 and 97 measure the amount of current flowing through the primary winding segments 108, 110 and 112,114 of the power output transformers 104 and 106, respectively. The sensors 95 and 97 supply primary winding current sense signals 162 and 164 having magnitudes which represent the magnitudes of the current flowing in the primary windings of the transformers 104 and 106, respectively. The voltage sensors 92 and 94 supply the voltage sense signals 96 and 98 which have magnitudes that represent the respective magnitudes of the voltage applied across the primary winding segments 108, 110 and 112, 114 of the transformers 104 and 106, respectively.

Current sensors 166 and 168 are connected to the secondary windings 124 and 126 of the power output transformers 104 and 106 to measure the current of the heater power signals 77 and 79, respectively. The current sensors 166 and 168 supply secondary or output current sense signals 170 and 172 having magnitudes which represent the magnitudes of the current of the heater power signals 77 and 79.

The primary current sense signals 162 and 164 are applied to peak current detectors 174 and 176, respectively, and the secondary current sense signals 170 and 172 are applied to peak current detectors 178 and 180, respectively. The peak current detectors 174-180 are each conventional and include conventional peak hold circuitry to detect and hold the highest or peak magnitude of any signal applied to the peak hold circuits, until the peak current detectors are reset. The peak current detectors 174, 176, 178 and 180 hold the peak magnitudes of the current signals 162, 164, 170 and 172, respectively, as peak magnitude current signals 162', 164', 170' and 172', until reset. The peak magnitude current signals 162', 164', 170' and 172' therefore represent the peak magnitudes of the current sense signals 162, 164, 170 and 172 during a sampling period of the detectors 174-180, respectively.

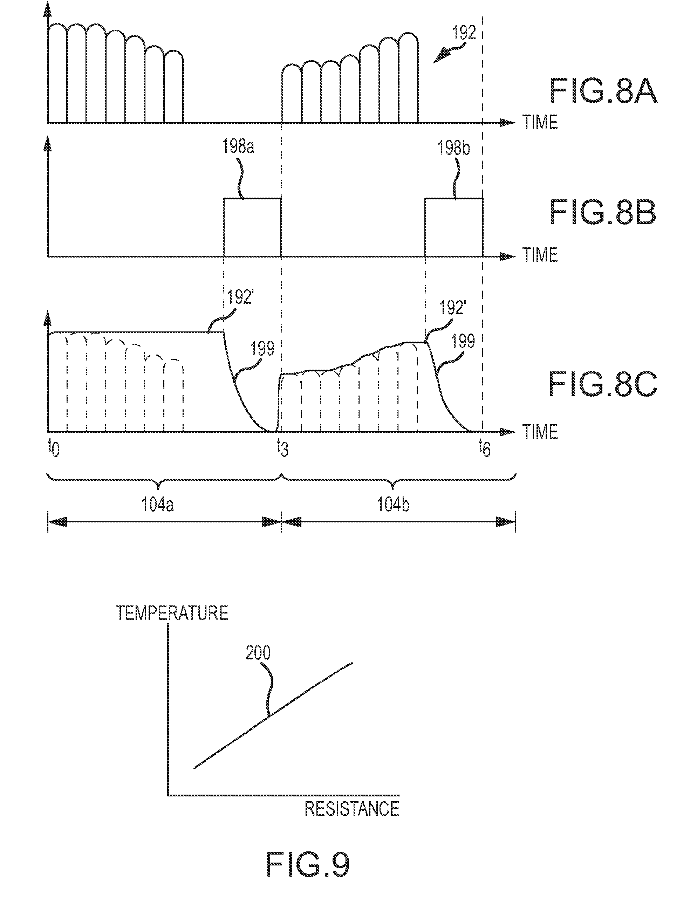

The sampling periods of the peak current detectors 174-180 are established by reset signals 182 and 184 which are asserted by the monitor and control processors 64 and 62 respectively. The reset signal 182 is asserted to the peak current detectors 174 and 176, and the reset signal 184 is asserted to the peak current detectors 178 and 180. The reset signals 182 and 184 (comparable to the reset signals 198a and 198b, Fig. 8B) are asserted once during each control cycle period 104 (Figs. 7A-7H), to assure that the peak current values 162', 164', 170' and 172' of the current conducted during that control cycle are obtained for use by the control and monitor processors 62 and 64 in regulating the output power and in controlling and monitoring the functionality of the energy source 26.

The peak magnitude current signals 170' and 172' are supplied to an analog to digital converter (ADC) 186. As shown in Fig. 6, the ADC 186 is an internal component of the control processor 62; however, the ADC 186 could also be a separate external component of the control processor 62. The ADC 186 converts the analog values of the peak current signals 170' and 172' to corresponding digital values at sampling points within each control cycle period 104. The sampling points are determined by a sequencer 188, which generally controls the sequence of all functions performed by the control processor 62, including supplying the converted peak digital values 170' and 172' of the corresponding analog peak current signals 170 and 172 to other routines executed by the control processor 62. The monitor processor 64 and the handpiece processor 66 also have ADCs and sequencers (neither shown) which operate in a similar manner to the ADC 186 and the sequencer 188 of the control processor 62.

Voltage sense signals 190 and 192 represent the voltages across the jaw heating elements 30 and 32, respectively. The voltage sense signals 190 and 192 are supplied to peak voltage detectors 194 and 196 within the handpiece 12. The peak voltage detectors 194 and 196 are conventional and include circuitry which detects and holds the maximum or peak value of the voltage sense signals 190 and 192 until the peak voltage detectors 194 and 196 are reset. The detectors 194 and 196 supply peak voltage signals 190' and 192' to the handpiece processor 66. The peak voltage signals 190' and 192' correspond to the peak or maximum values of the analog voltage sense signals 190 and 192 over a sampling period of the peak voltage detectors 194 and 196. The sampling period of the peak voltage detectors 194 and 196 is established by a reset signal 198 (198a, 198b, Fig. 8B) asserted by the handpiece processor 66. The reset signal 198 is asserted once during each control cycle 104 (Figs. 7A-7H), to assure that the peak values of the voltages applied to the jaw heating elements 30 and 32 during that control cycle are obtained for use in controlling and monitoring the functionality of the energy source 26.

The peak detectors 174, 176, 178, 180, 194 and 196 all operate in similar manner. The following description of peak detector functionality is presented in reference to exemplary signals shown in Figs. 8A-8C applied to the peak voltage detector 196. The voltage sense signal 192 is shown in Fig. 8A as having a variable magnitude over two control cycles 104a and 104b. Each voltage sense signal 192 is formed by four positive half-cycles of the heater power signal 79 and four negative half-cycles of the heater power signal 79 (Fig. 7H). The positive and negative pulses of the heater power signal are rectified into positive values as shown in Fig. 8A by a conventional rectifying capability of the peak detector 196. The rectifying capability assures that the maximum value of both the positive and negative half-cycles of the heater power signal 79 are detected and held. The first cycle period 104a starts at time to and ends at time t₃. The second cycle period 104b starts at time t₃ and ends at time t₆. Reset signals 198a and 198b are shown in Fig. 8B as asserted prior to times t₃ and t₆, prior to the start of both control cycles 104a and 104b. The assertion of the reset signals 198a and 198b cause the peak values 192' which are being held to dissipate or discharge as shown 199.

The peak voltage signal 192', shown in Fig. 8C, begins at a value which relates to the magnitude of the voltage sense signal 192 immediately after the reset signal has been de-asserted to the peak voltage detector 196. Sampling the peak voltage signal 192' begins at the start of the control cycle 104a and the maximum sampled magnitude for the duration of the first cycle period 104a is held until the reset signal 198a is asserted. The magnitude of the voltage sense signal 192 was near its maximum at the beginning of the control cycle 104a, as shown in Fig. 8C. When the reset signal 198a is de-asserted at time t₃ at the beginning of the second control cycle 104b, the magnitude of the voltage sense signal 192 has decreased compared to the magnitude of the voltage sense signal 192 shortly after time to. Consequently, the initial value of the peak voltage signal 192' at the beginning of the control cycle 104b starts low, but the magnitude of the peak voltage sense signal 192' continues to increase during the control cycle 104b, until heater power signal 79 (Fig. 7H) is no longer delivered when the gate control signal 136 is no longer asserted (Figs. 6 and 7F). Thus, the continually increasing value of the peak voltage signal 192' during the cycle period 104b illustrates that each peak detector will increase the magnitude of its peak output signal whenever its input signal increases above a previous value, until reset.

The control processor 62 uses the peak voltage values 190' and 192' along with the peak current values 170' and 172' to individually calculate resistance values of the jaw heating elements 30 and 32 during each control cycle period 104. The control processor 62 obtains the peak current values 170' and 172' by sampling the peak current detectors 178 and 180 during each control cycle period 104. The control processor 62 obtains the voltage values across the heating elements 30 and 32 by issuing commands to the handpiece processor 66 requesting the peak voltage values 190' and 192' derived by the peak voltage detectors 194 and 196.

The control processor 62 calculates the resistance of each of the jaw heating elements 30 and 32 during each control cycle 104 by dividing the peak voltage values 190' and 192' for each jaw heating element 30 and 32 by the peak current values 170' and 172', respectively. The calculated resistance value is thereafter used to determine the temperature of each jaw heating element. The correlation between resistance value and temperature of each jaw heating element is obtained from the known temperature coefficient characteristic relationship between temperature and resistance of the material which forms each jaw heating element 30 and 32. Graph 200, shown in Fig. 9, illustrates an exemplary positive temperature coefficient and resistance relationship. The graph 200 illustrates that for each resistance of each jaw heating element, that heating element is experiencing a single temperature. By knowing the resistance, obtained from dividing the peak voltage value by the peak current value, the corresponding temperature of the jaw heating element is obtained.

The graph 200 can be defined by an equation or by a lookup table. In either case, the equation or lookup table is stored in the memory 75 of the handpiece 12 (Fig. 5). A separate equation or lookup tables stored in the handpiece memory 75 allows the data to be calibrated to the exact characteristic relationship of temperature and resistance of each jaw heating element 30 and 32 specifically used in each handpiece 12. The equation or the data from the lookup table in the memory 75 of the handpiece is sent to the control processor 62 over the communication bus 68 by the handpiece processor 66 when the handpiece 12 is initially connected to the energy source 26. In this manner, the temperature determinations are specific to the individual resistance characteristics of each jaw heating element 30 and 32.

The ability to control the level of voltage from each variable voltage power supply 84 and 86 allows that voltage to be increased or decreased to compensate for manufacturing variances and slight variations in resistance of the jaw heating elements 30 and 32. In the event that one of the jaw heating elements 30 or 32 has a higher or lower resistance value than expected, the voltage from the power supply 80 is increased or decreased to ensure the same power is simultaneously delivered to each jaw heating element 30 and 32. Prior to performing a thermal tissue operation, and periodically during the procedure, the control processor 62 calculates resistance values for the jaw heating elements 30 and 32 and then signals the variable voltage power supplies 84 and 86 to adjust the voltage supplied, so that an equivalent and desired amount of power is delivered to each jaw heating element.

The level of voltage supplied from the variable voltage power supplies 84 and 86 to each jaw heating element 30 and 32 is calculated as the square root of the product of the desired power consumption of the jaw heating element at a particular time in one of the temperature versus time profiles 36 or 36' (Figs. 2A or 2B), 37 (Fig. 3) or 46 (Fig. 4) and the calculated resistance value of that jaw heater. Varying the voltage supplied to the jaw heating elements 30 and 32 in this manner ensures that equivalent amounts of electrical power are supplied to each of the jaw heating elements 30 and 32 despite the jaw heating elements 30 and 32 having different resistance values.

Varying the voltages of the variable voltage power supplies 84 and 86 is not used to regulate the temperature of the jaw heating elements 30 and 32 as part of the temperature feedback control. Instead, the temperatures of the jaw heating elements 30 and 32 are independently regulated by varying the average amount of current supplied to each of the jaw heating elements 30 and 32. The temperature of each of the jaw heating elements 30 and 32 is separately determined from the separately calculated resistance values, as explained above. These calculated temperatures are used in a feedback control algorithm by the control processor 62 to allow individual control over each of the heater power signal 77 and 79 to individually establish, maintain and regulate the temperature of each jaw heating element 30 and 32. Using resistance to temperature data (Fig. 9) that is particular to each jaw heating element 30 and 32 ensures that the derived temperature is accurate, thereby allowing closer regulation of the temperature during the thermal tissue operations.

Positioning the peak voltage detectors 194 and 196 within the handpiece 12 (Fig. 6) close the jaw heating elements 30 and 32 ensures that the voltage sense signals 190 and 192 and the corresponding peak voltage signals 190' and 192' are accurate by avoiding measurements that are degraded by the inherent voltage drop resulting from conducting the current of heater power signals 77 and 79 through the conductors of the cable 28 to the jaw heating elements 30 and 32 of the handpiece 12. Current flowing in a closed circuit path is the same at any point along the path, so the position of the current sensors 166 and 168 at the secondary windings 124 and 126 of the transformers 104 and 106 respectively, accurately represents the amount of current supplied to the jaw heating elements 30 and 32.

Some slight amount of power is inherently consumed by the transformers 104 and 106, so the amount of power delivered to the jaw heating elements 30 and 32 calculated by the control processor 62 in multiplying the peak values 170' and 172' of the secondary current sense signals 170 and 172 by the peak voltage signals 190' and 192' is slightly different from the value of the power calculated by the monitor processor 64 in multiplying the peak values 162' and 164' of the primary current sense signals 162 and 164 by the value of the primary voltage sense signals 96 and 98. Nonetheless, the comparative relationship of the power value calculated by the control processor 62 and the power value calculated by the monitor processor 64 allow the monitor processor 64 to determine whether the control processor 62 is performing appropriately under the circumstances.

The total amount of electrical energy supplied to each jaw heating element since the start of a thermal tissue operation to the end of that thermal tissue operation is calculated by adding the sum of electrical powers calculated multiplied by the time the power is delivered during each control cycle which has occurred since activation of the energy source 26 to accomplish that thermal tissue operation.

Reliable and intended operation of the thermal tissue operating system 10 is confirmed by a number of self-tests preferably conducted immediately after the energy source 26 is initially powered on. Such tests constitute power on self-tests (POSTs). Some of the individual self-tests are of both the energy source 26 and the handpiece 12. Some of the individual self-tests may be conducted without a handpiece 12 connected to the energy source 26. The control processor 62 coordinates the self-tests, and the monitor processor 64 makes independent determinations during the self-tests. Generally, both the monitor processor 64 and the control processor 62 independently determine whether or not one of the self-tests is successful. A particular self-test is considered to have failed if either or both of the control and monitor processors 62 and 64 determine that the self-test has failed. If either processor 62 or 64 determines that one of the self-tests has failed, the monitor processor 64 deactivates the relays 142 and 144 (Fig. 6) to prevent the delivery of the heater power signals 77 and 79 to the jaw heating elements 30 and 32 of the handpiece 12. In this manner, the handpiece 12 cannot be used in a surgical thermal procedure under conditions where there is a discrepancy or malfunction. Error messages or other alerts are issued on the display 54 and/or through the speaker 56 (Fig. 1). In this manner, the need to replace or service the energy source 26 or to replace the handpiece 12 is communicated to the user.

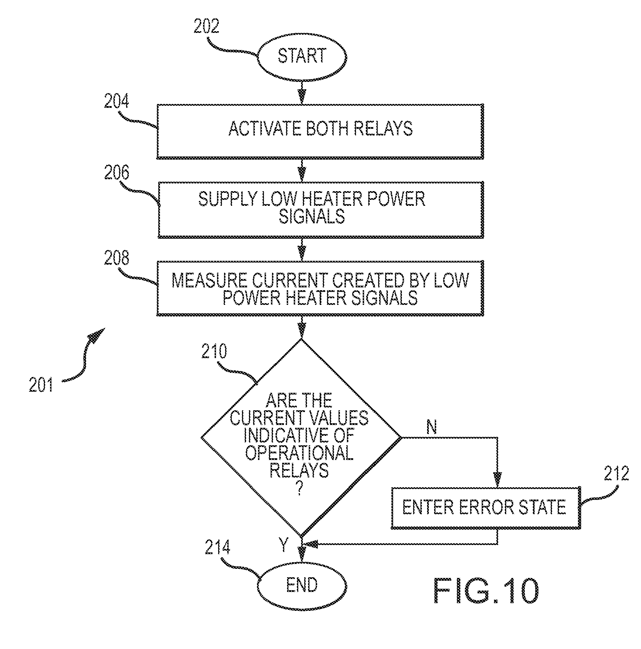

One of the self-tests of the energy source 26 is a relay test, described below in conjunction with Figs. 6 and 10. The relay test verifies that the relays 142 and 144 are operating as expected, thereby confirming that power delivery can be terminated to the handpiece 12 if an error or other unexpected condition develops during use of the thermal tissue operating system 10. The relay test involves activating the relays 142 and 144, supplying the heater power signals 77 and 79 through the relays 142 and 144, and verifying the presence or absence of current flowing through the relays 142 and 144 to indicate that the relays are in fact activated or deactivated as intended.

An exemplary process flow 201 for testing the relays 142 and 144 is shown in Fig. 10. The process flow 201 is jointly executed by the control processor 62 and the monitor processor 64 in a coordinated manner. The process flow 201 starts at 202. At 204, the monitor processor 64 asserts the relay activation signals 146 and 148, which causes the relays 142 and 144 to be activated. The control processor 62 then asserts relatively low duty cycle gate control signals 134 and 136 at 206. The relatively low duty cycle gate control signals 134 and 136 create relatively low power heater power signals 77 and 79. The relatively low power heater power signals 77 and 79 are used during the relay test to avoid elevating the temperature of the jaw heating elements 30 and 32 significantly enough to have a tissue heating effect. In this manner, the temperature of the jaw heating elements 30 and 32 in the jaws 14 and 16 (Fig. 1) will not burn or otherwise hurt any surgical personnel who might accidentally touch the jaws 14 and 16 during the test.

The current of the heater power signals 77 and 79 is then measured at 208. The current is measured by the monitor processor 64 sampling the peak current signals 162' and 164' from the peak current detectors 174 and 176. The peak current signals 162' and 164' represent the current of the primary alternating current signals 138 and 140 applied to the primary windings of the power output transformers 104 and 106. The current of the heater power signals 77 and 79 is measured by the control processor 62 sampling the peak current values 170' and 172' from the peak current detectors 178 and 180. The peak current values 170' and 172' are obtained from the current supplied to the jaw heating elements 30 and 32. Because there are losses in the transformers 104 and 106, and because the peak current values 170' and 172' utilized by the control processor 62 will be somewhat different from the peak current values 162' and 164' obtained by the monitor processor 64, differences in values within a predetermined range are deemed acceptable.

At 210, a determination is made of whether the different peak current values 170', 172' and 162', 164' indicate that the relays 146 and 148 are operating as intended. There are two different sets of peak current values which indicate whether the relays are operating as intended. The first set occurs when the handpiece 12 is not connected to the energy source 26. The second set occurs when the handpiece 12 is connected to the energy source 26. The peak current values will be different according to these two different situations. In the first situation when the handpiece 12 is not connected to the energy source 26, the expected peak current values will be very close to zero. In the second case when the handpiece 12 is connected to the energy source 26, the expected peak current values will be near the expected voltage values divided by the expected resistances of the jaw heating elements. The resistance values of the simulation heating elements 150 and 152 are different enough from the resistance values of the jaw heating elements 30 and 32 so that the processors 62 and 64 can distinguish between current flowing through each type of heating element. The determination at 210 is affirmative when the current values are within predetermined tolerances of the expected values.

If one or both of the relays 142 and 144 fails to operate when it is activated by the relay activation signals 146 and 148, the current from the secondary windings 124 and 126 of the applicable transformers 104 and 106 connected to the failed relay(s) will be directed to the simulation heating elements 150 and 152. The peak current values 170' and 172' from the failed relay(s) 142 and 144 will fall within a range that indicates the current is being conducted through the simulation heating element(s) 150 and 152, instead of the expected value of very near zero (when handpiece is not connected) or within the range of expected values that indicates the current is passing through one of the jaw heating elements 30 or 32 (when a handpiece is connected).

If the determination at 210 is negative, indicating that at least one of the relays 142 and 144 is not operating as intended, as discussed above, the process flow 201 progresses to 212 where the energy source 26 is placed into an error state. While in the error state, the energy source 26 communicates information related to the error to the user through the display 54 and speaker 56 (Fig. 1). If the determination at 210 is affirmative, indicating intended operation of the relays 142 and 144, the process flow 201 ends at 214. Ending the process flow 201 at 214 may be accompanied by communicating information of proper functionality to the user through the display 54 and the speaker 56 (Fig. 1).

It is important to verify that the relays 142 and 144 are operational and functioning as intended to ensure that the heater power signals 77 and 79 are directed to the jaw heating elements 30 and 32 when desired, or diverted to the simulation heating elements 150 and 152 should an error condition occur. Whenever an error state is entered, e.g. at 212, the relays 142 and 144 are preferably deactivated to prevent power delivery to the handpiece. Of course, diverting the heater power signals 77 and 79 to the simulation heating elements 150 and 152 avoids the potential of unintended power delivery to the handpiece 12.

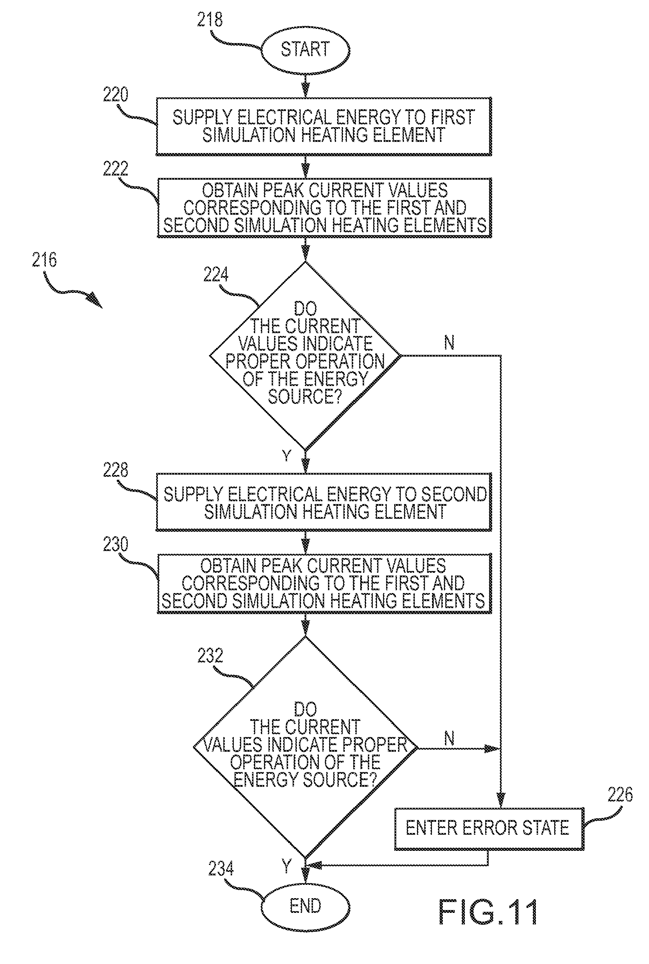

Another one of the self tests is a power test of the energy source 26. The power test verifies the correct and independent operation of the power generating and control functionality of the energy source 26, without requiring the handpiece 12 to be connected to the energy source 26. An exemplary process flow 216 for the power test is shown in Fig. 11 and described in connection with Fig. 6.

The process flow 216 is jointly executed by the control processor 62 and the monitor processor 64 in a coordinated manner. The relays 142 and 144 are kept in the deactivated state throughout the duration of the process flow 216. The power test process flow 216 starts at 218. At 220, the control processor 62 asserts a gate control signal 134 to the switches 116 and 118 to cause them to supply the heater power signal 77, while deasserting the gate control signal 136 to the switches 120 and 122. The heater power signal 77 should be conducted through the relay 142 to the simulation heating element 150. Then at 222, measurements of the peak current flowing through both of the simulation heating elements 150 and 152 are obtained by the control and monitor processors 62 and 64 from the peak current detectors 174, 176, 178 and 180.

A determination is then made at 224 whether the current values obtained at 222 indicate proper operation of the energy source 26. Proper operation is indicated if the peak current values read by the control and monitor processors reveal that a predetermined amount of current is flowing through the relay 142 to the simulation heating element 150 and that no current is flowing through the relay 144 to the simulation heating element 152. If these conditions are satisfied, the determination at 224 is affirmative. If either one of these conditions is not satisfied, the determination at 224 is negative, indicating a problem with the power generating or relay control capability of the energy source 26.

A negative determination at 224 causes the process flow 216 to progress to 226 where the energy source 26 is placed in an error state and appropriate indications of a problem are provided to the user through the display 54 and speaker 56 (Fig. 1). If the determination at 224 is affirmative, then the process flow 216 continues to 228.

At 228, the control processor 62 asserts a gate control signal 136 to the switches 120 and 122 to cause them to supply the heater power signal 79, while deasserting the gate control signal 134 to the switches 116 and 118. The heater power signal 79 should be conducted through the relay 144 to the simulation heating element 152. Then at 230, measurements of the peak currents flowing through both of the simulation heating elements 150 and 152 are obtained by the control and monitor processors 62 and 64 from the peak current detectors 174, 176, 178 and 180.

A determination is then made at 232 whether the current values obtained at 230 indicate proper operation of the energy source 26. Proper operation is indicated if the peak current values read by the control and monitor processors indicates that a predetermined amount of current is flowing though the relay 144 to the simulation heating element 152 and that no current is flowing through the relay 142 to the simulation heating element 150. If both of these conditions are satisfied, the determination at 232 is affirmative. If either of these conditions is not satisfied, the determination at 232 is negative, indicating a problem with the power generating or relay control capability of the energy source 26.

A negative determination at 232 causes the process flow 216 to progress to 226 where the energy source 26 is placed in an error state and appropriate indications of a problem are provided to the user through the display 54 and speaker 56 (Fig. 1). If the determination at 232 is affirmative, then the process flow 216 terminates at 234, indicating that the power test has shown satisfactory performance of the energy source 26.

The power test verifies the capability of the energy source 26 to deliver power without requiring the handpiece 12 to be connected to the energy source 26 and without delivering electrical energy to heat the jaw heating elements 30 and 32. The power test also verifies that there is no leakage current in the current conducting paths to the separate jaw heating elements, as well as proper individual control over the switches 116, 118 and 120, 122.

Another self-test of both the energy source 26 and the handpiece 12 is a peak detector test. The peak detector test is employed to test the peak current detectors 174-180 of the energy source 26 and to test the peak voltage detectors 194 and 196 of the handpiece 12 (Fig. 6). The peak detector tests are executed by the control, monitor and handpiece processors 62, 64 and 66. The purpose of each peak detector test is to verify that each peak detector maintains or holds its peak values, without degradation of those values, over the sampling period. Ensuring that the peak signals are maintained as expected until reset at the end of each sampling period (Fig. 8C) is important because the feedback pulse width modulation power control regulation 101 executed by the control routine 103 of the control processor 62 is based on the accuracy of the peak signals.

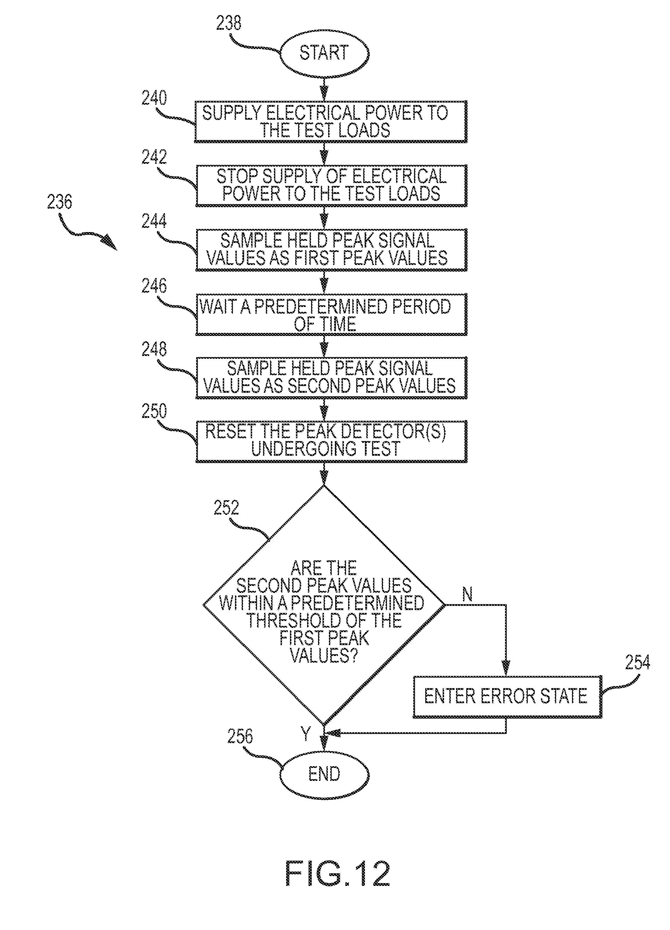

An exemplary process flow 236 for testing each of the peak detectors 174-180, 194 and 196, is shown in Fig. 12 and described in conjunction with Fig. 6. The process flow 236 is jointly executed by the control and monitor processors 62 and 64 when testing each of the peak current detectors 174-180 and is additionally executed by the handpiece processor 66 when testing each of the peak voltage detectors 194 and 196. Each peak detector can be tested separately from the other peak detectors. However, the peak current detectors 174, 176, 178 and 180 can be tested simultaneously, because the tests of the peak current detectors 174-180 are performed using the load simulation heating elements 150 and 152. The peak voltage detectors 194 and 196 can also be tested simultaneously, because the test of the peak detectors 194 and 196 are performed using the jaw heating elements 30 and 32. The simultaneous tests of the peak current detectors 174-180 should be performed separately from the simultaneous tests of the peak voltage detectors 194 and 196.

The process flow 236 for each peak detector starts at 238. At 240, electrical energy is supplied to test loads. The test loads for testing the peak current detectors 174-180 are the simulation heating elements 150 and 152. The test loads for testing the peak voltage detectors 194 and 196 are the jaw heating elements 30 and 32 of the handpiece 12. The relays 142 and 144 are deactivated to conduct the resulting heater power signals 77 and 79 to the simulation heating elements 150 and 152 for the peak current detector tests. The relays 142 and 144 are activated to conduct the heater power signals 77 and 79 to the jaw heating elements 30 and 32 for the peak voltage detector tests. The gate control signals 134 and 136 are applied to the enabled oscillators 128 and 129 to create electrical energy (heater power signals 77 and 79) applied to the test loads.

After the applicable sense signal 162, 164, 170, 172, 190 or 192 has been delivered to each peak detector 174-180, and 194 and 196 undergoing test, the supply of the electrical energy (heater power signals 77 and 79) to the test load connected to each peak detector undergoing test is terminated at 242. The supply of electrical energy is terminated by the deassertion of the gate control signals 134 and 136 to the oscillators 128 and 129. As a result, the magnitude of the electrical energy immediately goes to zero.

Immediately thereafter at 244, the processors sample or read the peak signal 162', 164', 170', 172', 190' or 192' held by each peak detector undergoing the test. Each sample value is obtained immediately after the terminating the delivery of the electrical energy to the test loads, and each immediately obtained sample value constitutes a first peak value. The first peak value supplied by each peak current detector 178 and 180 is obtained by the control processor 62. The first peak value supplied by each peak current detector 174 and 176 is obtained by the monitor processor 64. The first peak value supplied by each peak voltage detector 194 and 196 is obtained by the handpiece processor 66 and is communicated to the control processor 62 over the communication bus 68.

A predetermined amount of time which is greater than the duration of a control cycle 104 (Figs. 7A-7H and 8A-8C) is then allowed to elapse, as indicated at 246. The predetermined amount of time which is allowed to elapse is designated as a sample interval. The peak signal held by each peak detector undergoing the test is again sampled at 248, at the end of the sample interval. The sampling which occurs at 248 is the same type of sampling as was previously described at 244. The value of each signal sampled at 248 at the end of the sample interval constitutes a second peak value. Each second peak value is derived from the value of the signal 162', 164', 170', 172', 190' or 192' held by each peak detector 174, 176, 178, 180, 194 and 196 undergoing the test, after the sample interval has elapsed. Each peak detector undergoing the test is then reset (Fig. 8B), at 250.

If the peak detector undergoing the test is performing correctly, each second peak value sampled at 248 should remain at approximately the same value as each corresponding first peak value sampled at 244. If the first and second peak values differ by a significant amount, abnormal operation of the tested peak detector is indicated. Proper functionality of the peak detector undergoing the test is established by a range of predetermined values of the difference between the first and second peak values. A relatively larger range indicates abnormal functionality, while a relatively smaller range indicates acceptable functionality.

To determine whether each peak detector is operating correctly, the second peak value is compared to the first peak value to determine whether the second peak value is within a predetermined threshold or range of the corresponding first peak value, as shown at 252. If the determination at 252 is negative, indicating that the peak detector undergoing the test is experiencing problematic functionality, the process flow 236 progresses to 254 where the energy source 26 enters the error state and appropriate indications of the problematic peak detector are provided to the user through the display 54 and speaker 56 (Fig. 1). If the determination at 252 is affirmative, indicating that the peak detector undergoing the test is operating properly, the process flow 236 ends at 256, indicating that each tested peak detector has shown satisfactory performance. The process flow 238 is repeated as necessary to verify that all the peak detectors are operating properly or to indicate any peak detector presenting problematic functionality.

An additional self-test to verify the functionality and integrity of the jaw heating elements 30 and 32 is described in the above referenced US patent application US/SN 12,842,659. Unlike the self-tests described herein, this additional test is performed continuously during the use of the thermal tissue operating system 10 rather than occasionally or initially on powering on the energy source 26. This additional test is referred to herein as a jaw heating element integrity test. The jaw heating element integrity test verifies that the jaw heating elements 30 and 32 are receiving electrical power from the energy source 26 and that the calculated resistances of the jaw heating elements remain within predetermined expected ranges during the course of the surgical procedure in which the thermal tissue operating system 10 (Fig. 1) is employed. Verifying that the resistances of the jaw heating elements 30 and 32 remain within predetermined expected ranges identifies problems with the jaw heating elements that could adversely affect the feedback power regulation capability, the amount of power delivered to the jaw heating elements 30 and 32, the temperature of the jaw heating elements 30 and 32, and/or the ability of the thermal tissue operating system to attain the desired time versus temperature profiles for the tissue sealing operation (Fig. 2), the tissue cutting operation (Fig. 3), and the combined tissue sealing and cutting operation (Fig. 4), among other things.

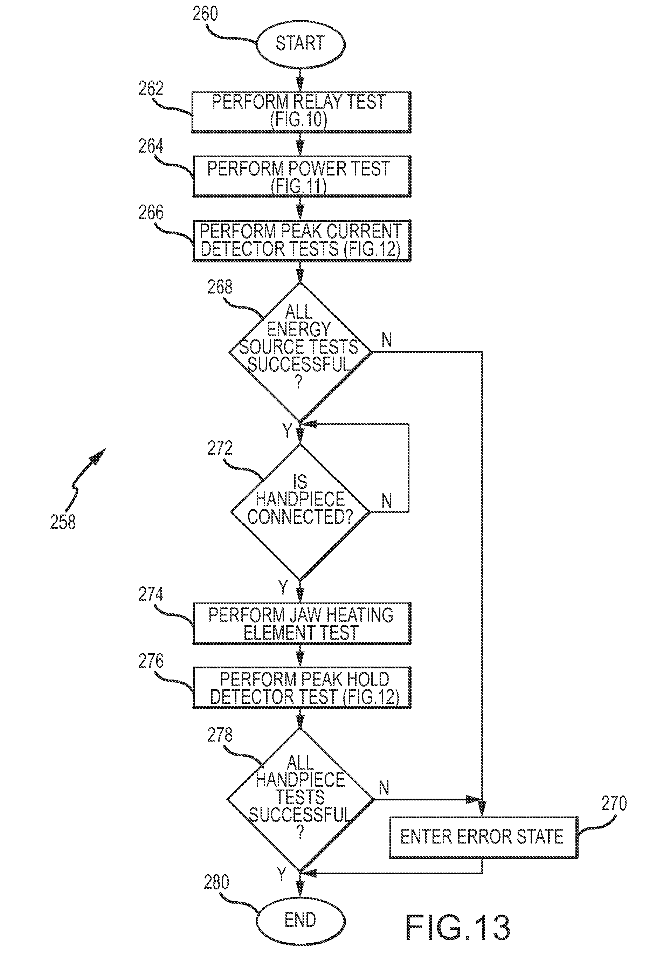

An exemplary power on self-test (POST) for the thermal tissue operating system 10, such as that shown at 258 in Fig. 13, preferably incorporates the relay test 201 (Fig. 10), the power test 216 (Fig. 11), the peak detector tests 236 (Fig. 12) and the jaw heating element integrity test (discussed in application US/SN 12/842,659). The process flow 258 is performed by the control processor 62 with assistance from the monitor processor 64 for the relay, power and peak current detector tests, and also with assistance from the handpiece processor 66 for the peak voltage detector and jaw heating element integrity tests.

The overall POST process flow 258 starts at 260. At 262, the energy source 26 performs the relay test described in the process flow 201 (Fig. 10). Then at 264, the energy source 26 performs the power test described in the process flow 216 (Fig. 11). The energy source 26 then, at 266, performs the peak detector tests described in the process flow 256 (Fig. 12) for the peak current detectors 174-180. At 268, the control processor 62 then determines whether all of the tests at 262, 264 and 266 have completed successfully without entering an error state. If the determination at 268 is negative, then the process flow 258 progresses to 270 where the energy source 26 enters into the error state and appropriate indications of the problem are provided to the user through the display 54 and speaker 56 (Fig. 1). If the determination at 268 is affirmative, then the process flow 258 progresses to 272.

At 272, the control processor 62 checks to determine whether or not the handpiece 12 is connected to the energy source 26. This determination is made by the control processor 62 continuously attempting to send a predefined query communication to the handpiece processor 66 over the communication bus 68 and waiting for a predefined response communication from the handpiece processor 66 that indicates that communication has been established with the handpiece processor 66 as a result of connecting the handpiece 12 to the energy source 26. If the determination at 272 is negative, the process flow 258 continuously loops back to 272 to perform the determination at 272 until the handpiece is connected. When the handpiece 12 is connected to the energy source 26, the handpiece processor 66 responds to the query thereby informing the control processor 62 that the handpiece 12 is connected to the energy source 26. The determination at 272 is then affirmative, and the process flow 258 progresses to 274.

At 274, the thermal tissue operating system 10 performs the jaw heating element integrity test described in application number US/SN 12/842,659 Then at 276, the thermal tissue operating system 10 performs the peak detector test 256 (Fig. 12) for the peak voltage detectors 194 and 196. The control processor 62 checks to determine whether the POST tests of the handpiece 12 performed at 274 and 276 completed successfully. If the determination at 278 is negative, then the process flow 258 progresses to 270 where the energy source 26 enters into the error state. If the determination at 278 is affirmative, or after entering the error state at 270, the process flow 258 ends at 280.

The POST test 258 is considered to have been successful if it completes without entering the error state at 270. If the process flow 258 enters the error state at 270, information related to the error, or failure of a particular test, is supplied to the display 54 and speaker 56 to alert the user. After the successful completion of the process flow 258, the thermal tissue operating system 10 is ready and waiting to perform thermal tissue operations in a surgical procedure.

The thermal tissue operating system 10 of the present invention is advantageous in many regards. The energy source 26 tests its own power generating capability by using the simulation heating elements 150 and 152 without requiring the handpiece 12 to be connected to the energy source 26. This power test performed using the simulation heating elements 150 and 152 avoids two problems associated with using the jaw heating elements 30 and 32 of the handpiece 12 to perform the power test. The first problem avoided is that in the event of an unsuccessful power test, it is usually unknown whether the failure occurred within the energy source 26 or within the handpiece 12. The second problem avoided is that using the jaw heating elements 30 and 32 as the load for the power test results in heating the jaw heating elements 30 and 32. Heating the jaw heating elements 30 and 32 when not used in a thermal tissue operation risks accidental injury to the surgeon or other surgical personnel who might touch the hot jaw heating elements 30 and 32.

Performing the peak detector tests helps ensure the accuracy of the voltage and current values used by the control processor 62 to calculate resistance values and the temperatures of the jaw heating elements 30 and 32. The control processor 62 is better able to attain, maintain and regulate the desired temperature of the jaw heating elements 30 and 32 according to the temperature versus time profiles (Figs. 2, 3 and 4) when the voltage and current values accurately reflect the resistance, voltage and current at, across or through the jaw heating elements 30 and 32.

Confirming proper operation of the relays 142 and 144 gives the assurance that the electrical power can be directed to the simulation heating elements 150 and 152, should an unexpected operating condition develop during use.

Presently preferred embodiments of the present invention and many of its improvements have been described with a degree of particularity. This description is of preferred examples of implementing the invention, and is not necessarily intended to limit the scope of the invention. The scope of the invention is defined by the following claims.

## Claims

1. An electrical energy source for a thermal tissue operating system which also which also includes a handpiece that connects to the energy source, the handpiece including a pair of opposing jaws which contact and compress tissue during a thermal tissue operation, at least one of the jaws including a jaw heating element for converting electrical power into thermal heat energy applied in the thermal tissue operation, the energy source creating a heater power signal having a voltage and current, the energy source supplying voltage and current from the heater power signal to each jaw heating element during the thermal tissue operation, the energy source further comprising:
- a simulation heating element;
- a controllable switch connected to the jaw heating element and to the simulation heating element, the controllable switch having a deactivated state which conducts voltage and current from the heater power signal to the simulation heating element and an activated state which conducts voltage and current from the heater power signal to the jaw heating element;
- a controller which controls the controllable switch to assume the activated and deactivated states; and
- a sensor connected to sense one of the voltage or the current of the
heater power signal and to supply a sense signal related thereto;
and wherein the controller is operative to receive the sense signal, to control the controllable switch into the deactivated state and to determine whether the controllable switch is correctly in the deactivated state from the sense signal.

2. An energy source as defined in claim 1, wherein:
- the controllable switch conducts the output power waveform to the simulation heating element in the deactivated state;
- the controllable switch conducts the heater power signal to the jaw heating element in the activated state;
- the first aforesaid sensor constitutes a first sensor;
- the sense signal from the first sensor constitutes a first sense signal; and further comprising a second sensor connect to sense one of the voltage or the current of a signal used to create the heater power signal and to supply a related second sense signal;
and wherein the controller is operative to also receive the second sense signal and to determine whether the controllable switch is correctly in the activated and deactivated states from the first and second sense signals.

3. An energy source as defined in claim 2, wherein each jaw of the handpiece includes a jaw heating element, and a first jaw heating element is associated with one jaw and a second jaw heating element is associated with the other jaw, and wherein:
- the first aforesaid simulation heating element constitutes a first simulation heating element;
- the first aforesaid controllable switch constitutes a first controllable switch;
- the first controllable switch is connected to the first jaw heating element and to the first simulation heating element;
- the deactivated state of the first controllable switch conducting the heater power signal to the first simulation heating element, and the activated state of the first controllable switch conducting the heater power signal to the first jaw heating element;
and further comprising:
- a second simulation heating element;
- a second controllable switch connected to the second jaw heating element and to the second simulation heating element, the second controllable switch having a deactivated state which conducts the heater power signal to the second simulation heating element and an activated state which conducts voltage and current from the heater power signal to the second jaw heating element;
and wherein:
- the first and second since signals are associated with each of the first and second controllable switches;
- the controller controls both the first and second controllable switches to assume the activated and deactivated states; and
- the controller is further operative to determine whether the first and second controllable switches are each correctly in the activated and deactivated states from the first and second sense signals associated with each of the first and second controllable switches.

4. An energy source as defined in claim 3, wherein:
- the controller comprises a control processor and a monitor processor;
- the control processor is connected to receive the first sense signal;
- the monitor processor is connected to receive the second sense signal;
- the control processor and the monitor processor each independently determine whether the first and second controllable switches are each correctly in both the activated and deactivated states from the sense signals received by the control and monitor processors; and
- at least one of the control processor or the monitor processor operatively controls the first and second controllable switches into the deactivated state upon either one of the control processor and the monitor processor determining that either one of the first and second controllable switches is not correctly in the activated or deactivated states.

5. An energy source as defined in claim 3 in combination with the handpiece, wherein:
- the controller comprises a control processor and a monitor processor in the energy source and a handpiece processor in the handpiece;
- the control processor is connected to receive the first sense signal;
- the monitor processor is connected to receive the second sense signal;
- the handpiece processor is operative to the determine a first voltage across the first jaw heating element and to determine a second voltage across the second jaw heating element and to communicate the first and second voltages to the control processor; and
- the control processor responds to the first sense signal and the first voltage to regulate the heater power signal to the first jaw heating element; and
- the control processor responds to the second sense signal and the second voltage to regulate the heater power signal to the second jaw heating element.

6. An energy source as defined in claim 2, wherein:
- at least one of the first or second sensors includes at least one peak hold detector operative over a sample time interval to detect and hold a peak value corresponding to a maximum value of one of the current or voltage of the signal sensed by that sensor over that sample time interval; and
- the controller is further operative to determine the peak value at a relatively early point in the sample time interval and to determine the peak value at a relatively late point in the sample time interval, to compare the determined relatively early and late peak values, to determine whether any difference between the relatively early and late peak values falls outside of a predetermined range which indicate insufficient functionality of that peak hold detector, and to issue an error indication when the difference between the relatively early and late peak values falls outside of the predetermined range.

7. An energy source as defined in claim 1 in combination with the handpiece, wherein:
- the jaw heating element is a resistor;
- the sensor senses the current of the heater power signal conducted by the jaw heating element resistor;
- the controller is operative to the determine a voltage across the jaw heating element resistor when the heater power signal is conducted through the jaw heating element resistor with the controllable switch in the activated state; and
- the controller is further operative to determine to determine a resistance value of the jaw heating element resistor from the current conducted by the jaw heating element resistor and the voltage across the jaw heating element resistor, and to control the controllable switch into the deactivated state upon the resistance value of the jaw heating element resistor falling outside of a predetermined range of resistances.

8. An energy source as defined in claim 1 in combination with the handpiece, wherein:
- the jaw heating element is a resistor;
- the sensor senses the current of the heater power signal conducted by the jaw heating element resistor;
- the controller is operative to the determine a voltage across the jaw heating element resistor when the heater power signal is conducted through the jaw heating element resistor with the controllable switch in the activated state;
- the controller is operative to determine to determine a resistance value of the jaw heating element resistor from the current conducted by the jaw heating element resistor and the voltage across the jaw heating element resistor;
- the controller is operative to determine the temperature of the jaw heating element resistor from the resistance value of the jaw heating element resistor; and
- the controller is further operative to regulate the power of the heater power signal conducted to the jaw heating element resistor in response to the temperature of the jaw heating element resistor determined by the resistance value.

9. A method of performing a test of a thermal tissue operating system which includes an energy source which produces electrical power and a handpiece which connects to the energy source and includes a pair of opposing jaws which compress tissue during a thermal tissue operation, at least one of the jaws including a jaw heating element for converting electrical power into thermal heat energy applied to the compressed tissue during the thermal tissue operation, the energy source including a controllable switch and a simulation heating element which simulates the jaw heating element, the controllable switch conducting the electrical power to the jaw heating element in one state when the handpiece is connected to the energy source, and the controllable switch conducting the electrical power to the simulation heating element in another state, the method comprising:
- establishing the one state of the controllable switch to conduct the electrical power to the simulation heating element;
- conducting the electrical power through the controllable switch to the simulation heating element when the controllable switch is in the one state;
- measuring one of the voltage or current of the electrical power conducted through the simulation heating element to obtain a measured value;
- referencing a predetermined range of expected values of the measured value indicative of normal voltage or current conducted through the simulation heating element; and
- communicating an error message when the measured value is not within the predetermined range of expected values.

10. A method as defined in claim 9, wherein each jaw of the handpiece includes a jaw heating element, the aforementioned jaw heating element constituting a first jaw heating element, the aforementioned controllable switch constituting a first controllable switch, the aforementioned simulation heating element constituting a first simulation heating element, the aforementioned measured value constituting a first measured value, and the aforementioned predetermined range of expected values constituting a first range of expected values for the first jaw element; the handpiece additionally comprising a second jaw heating element, the energy source additionally comprising a second controllable switch and a second simulation heating element, the second controllable switch conducting electrical power to the second jaw heating element in one state when the handpiece is connected to the energy source and conducting electrical power to the second simulation heating element in another state; the method further comprising:
- establishing the one state of the second controllable switch in which the electrical power is conducted to the second simulation heating element;
- conducting the electrical power through the second controllable switch to the second simulation heating element when the second controllable switch is in the one state;
- measuring one of the voltage or current of the electrical power conducted through the second simulation heating element to obtain a second measured value;
- referencing a predetermined range of expected values of the second measured value indicative of normal voltage or current conducted through the second simulation heating element; and
- communicating an error message when the second measured value is not within the predetermined range of expected values of the second measured value.

11. A method as defined in claim 10, further comprising:
- measuring current through the second simulation heating element while the electrical power is conducted only through the first simulation heating element, to obtain a third value;
- measuring current through the first simulation heating element while the electrical power is conducted only through the second simulation heating element, to obtain a fourth value; and
- communicating an error message when at least one of the third and fourth values is a finite value not substantially equal to zero.

12. A method as defined in claim 9, wherein the measured value is obtained by measuring current of the electrical power conducted through the simulation heating element; and the method further comprises:
- including in the predetermined range of expected values a first subset of values which indicates that a handpiece is connected to the energy source and a second subset of values which indicates that a handpiece is not connected to the energy source;
- communicating the error message when the measured value is not within the first subset of values and the handpiece is connected to the energy source; and
- communicating the error message when the measured value is not within the second subset of values and the handpiece is not connected to the energy source.

13. A method as defined in claim 9, further comprising:
- establishing a sample time interval;
- referencing a peak hold detector to detect a peak value of the measured value during the sample time interval and thereafter to hold the peak value;
- determining the peak value held by the peak hold detector at a relatively early point in the sample time interval after the peak value of the measured value has been detected;
- determining the peak value held by the peak hold detector at a relatively late point in the sample time interval after the peak value of the measured value has been detected;
- comparing the peak values determined at the relatively early and relatively late points in the sample time interval;
- referencing a predetermined threshold of variations in peak values held by the peak hold detector to define acceptable functionality of the peak hold detector; and
- communicating an error message when the peak values determined at the relatively early and relatively late points fall outside of the predetermined threshold.

14. A method as defined in claim 9, further comprising:
- conducting the electrical power through the controllable switch to the jaw heating element;
- deriving a peak voltage value from the maximum voltage applied to the jaw heating element during the sample time interval;
- deriving a peak current value from the maximum current conducted by the jaw heating element during the sample time interval;
- calculating a resistance value of the jaw heating element by dividing the peak voltage value by the peak current value;
- referencing a predetermined range of normal resistance values of the jaw heating element; and
- communicating an error message when the calculated resistance value is not within the predetermined range of normal resistance values.

15. A method of performing a test of a thermal tissue operating system which includes an energy source and a handpiece which connects to the energy source, the energy source supplying electrical power to a jaw heating element of the handpiece for conversion into thermal energy to heat tissue compressed by a jaw during a thermal tissue operation, the method comprising:
- including a simulation heating element in the energy source which simulates characteristics of the jaw heating element;
- supplying the electrical power to a simulation heating element of the energy source during the test;
- measuring an electrical characteristic of power conducted through the simulation heating element;
- referencing a predetermined range of expected values for the measured electrical characteristic; and
- communicating an error message when the measured electrical characteristic is not within the predetermined range of expected values.
